# EUROPÄISCHE PATENTSCHRIFT

(11) **EP 2 326 955 B1**
(45) Veröffentlichungstag und Bekanntmachung des Hinweises auf die Patenterteilung: **26.02.2014**
(21) Anmeldenummer: 09777830.2
(22) Anmeldetag: 12.08.2009
(51) Int. Cl.: G01N 33/533, G01N 33/58, C07K 1/13, C09K 11/77, G01N 21/64

(54) **VERFAHREN ZUR MARKIERUNG UND/ODER IDENTIFIZIERUNG VON BIOMOLEKÜLEN**
METHOD FOR MARKING AND/OR IDENTIFYING BIOMOLECULES
PROCÉDÉ DE MARQUAGE ET/OU D'IDENTIFICATION DE BIOMOLÉCULES

(30) Priorität: 19.09.2008 EP 08016549; 22.09.2008 DE 102008048353; 23.09.2008 DE 102008048605
(43) Veröffentlichungstag der Anmeldung: 01.06.2011
(73) Patentinhaber: Inbio Prof. Jürgen Büddefeld Dr. Peter Klauth Prof. Manfred Rietz Gbr, 47239 Duisburg (DE)
(72) Erfinder: KLAUTH, Peter, 41189 Mönchengladbach (DE)
(74) Vertreter: Von Rohr
(86) Internationale Anmeldenummer: PCT/EP2009/005846
(87) Internationale Veröffentlichungsnummer: WO 2010/031471

(56) Entgegenhaltungen:
- WO-A1-00/39335
- KNOPF H-P ET AL: "PREPARATION OF EUROPIUM STREPTAVIDIN IN A TIME-RESOLVED FLUOROIMMUNOASSAY FOR INTERLEUKIN-3" JOURNAL OF IMMUNOLOGICAL METHODS, ELSEVIER SCIENCE PUBLISHERS B.V.,AMSTERDAM, NL, Bd. 138, Nr. 2, 1. Januar 1991 (1991-01-01), Seiten 233-236, XP002299187 ISSN: 0022-1759
- YUAN J ET AL: "A NEW TETRADENTATE BETA-DIKETONATE-EUROPIUM CHELATE THAT CAN BE COVALENTLY BOUND TO PROTEINS FOR TIME-RESOLVED FLUOROIMMUNOASSAY" ANALYTICAL CHEMISTRY, AMERICAN CHEMICAL SOCIETY. COLUMBUS, US, Bd. 70, Nr. 3, 1. Januar 1998 (1998-01-01) , Seiten 596-601, XP002932638 ISSN: 0003-2700
- JINGLI YUAN ET AL: "Lanthanide Complex-Based Fluorescence Label for Time-Resolved Fluorescence Bioassay" JOURNAL OF FLUORESCENCE, KLUWER ACADEMIC PUBLISHERS-PLENUM PUBLISHERS, NE, Bd. 15, Nr. 4, 1. Juli 2005 (2005-07-01), Seiten 559-568, XP019281718 ISSN: 1573-4994
- LIM M J ET AL: "A Luminescent Europium Complex for the Sensitive Detection of Proteins and Nucleic Acids on Membrane Supports" ANALYTICAL BIOCHEMISTRY, ACADEMIC PRESS INC, NEW YORK, Bd. 245, 1. Januar 1997 (1997-01-01), Seiten 184-195, XP002131527 ISSN: 0003-2697
- WU F-B ET AL: "SYNTHESIS OF A HIGHLY FLUORESCENT [beta]-DIKETONE-EUROPIUM CHELATE AND ITS UTILITY IN TIME-RESOLVED FLUOROIMMUNOASSAY OF SERUM TOTAL THYROXINE" ANALYTICAL CHEMISTRY, AMERICAN CHEMICAL SOCIETY. COLUMBUS, US, Bd. 22, Nr. 74, 15. November 2002 (2002-11-15), Seiten 5882-5889, XP001141042 ISSN: 0003-2700
- YAO FENGJI ET AL: "A fluorescence quenching immunoassay method for determination of trace albumin using unlabeled europium chelate" CHINESE CHEMICAL LETTERS, INSTITUTE OF MATERIA MEDICA, CN, Bd. 3, Nr. 12, 1. Januar 1992 (1992-01-01) , Seiten 1011-1014, XP009124876 ISSN: 1001-8417
- CASTELLANO F N ET AL: "Long-lifetime Ru(II) complexes as labeling reagents for sulfhydryl groups" ANALYTICAL BIOCHEMISTRY, ACADEMIC PRESS INC, NEW YORK, Bd. 255, Nr. 2, 15. Januar 1998 (1998-01-15), Seiten 165-170, XP002480897 ISSN: 0003-2697
- TSARYUK V ET AL: "Effect of ligand radicals on vibrational IR, Raman and vibronic spectra of europium beta-diketonates" SPECTROCHIMICA ACTA. PART A: MOLECULAR AND BIOMOLECULAR SPECTROSCOPY, ELSEVIER, AMSTERDAM, NL, Bd. 61, Nr. 1-2, 1. Januar 2005 (2005-01-01), Seiten 185-191, XP004649448 ISSN: 1386-1425
- PENG CHUNYUN ET AL: "Synthesis, characterization, and luminescence properties of the ternary europium complex covalently bonded to mesoporous SBA-15." THE JOURNAL OF PHYSICAL CHEMISTRY. B 18 AUG 2005, Bd. 109, Nr. 32, 18. August 2005 (2005-08-18), Seiten 15278-15287, XP009124761 ISSN: 1520-6106
- Y. SHEN & BP. SULLIVAN: "A versatile preparative route to 5-substituted-1,10-phenantroline ligands via 1,10-phenantroline 5,6-epoxide" INORGANIC CHEMISTRY, Bd. 34, 1995, Seiten 6235-6236, XP002552985
- WILKINSON D: "A one-step fluorescent detection method for lipid fingerprints; Eu(TTA)3.2TOPO.", FORENSIC SCIENCE INTERNATIONAL 4 JAN 1999 LNKD- PUBMED:10069019, vol. 99, no. 1, 4 January 1999 (1999-01-04), pages 5-23, ISSN: 0379-0738
- ANDERSON J R ET AL: "EUROPIUM CHELATE AND FLUORESCENT BRIGHTENER STAINING OF SOIL PROPAGULES AND THEIR PHOTO MICROGRAPHIC COUNTING PART 1 METHODS", SOIL BIOLOGY AND BIOCHEMISTRY, vol. 7, no. 3, 1975, pages 205-210, ISSN: 0038-0717
- SCAFF W L JR ET AL: "Fluorescent europium chelate sta.", JOURNAL OF BACTERIOLOGY APR 1969 LNKD- PUBMED:4181107, vol. 98, no. 1, April 1969 (1969-04), pages 246-248, ISSN: 0021-9193

## Beschreibung

Die vorliegende Erfindung betrifft das Gebiet der Markierung und/oder Identifizierung von Biomolekülen, wie insbesondere Proteinen, Peptiden, Antikörpern und Nukleinsäuren.

Insbesondere betrifft die vorliegende Erfindung ein Verfahren zur Markierung und/oder Identifizierung von Biomolekülen, insbesondere Proteinen, Peptiden, Antikörpern und Nukleinsäuren.

Des weiteren betrifft die vorliegende Erfindung die Verwendung von Chelatkomplexen von Elementen der Seltenen Erden zur Markierung und/oder Identifizierung von Biomolekülen, insbesondere Proteinen, Peptiden, Antikörpern und Nukleinsäuren.

Gleichermaßen betrifft die vorliegende Erfindung die Verwendung von Chelatkomplexen von Elementen der Seltenen Erden zur Fluoreszenzmarkierung und/oder -identifizierung von Biomolekülen, insbesondere Proteinen, Peptiden, Antikörpern und Nukleinsäuren.

Weiterhin betrifft die vorliegende Erfindung ein Kit zur Markierung und/oder Identifizierung mindestens eines Biomoleküls.

Schließlich betrifft die vorliegende Erfindung ein Biomolekül/Seltenerdkomplex-Konjugat, welches durch Inkontaktbringen bzw. Umsetzung mindestens eines Biomoleküls einerseits und mindestens eines Seltenerdkomplexes andererseits erhältlich ist.

Die aus dem Stand der Technik bekannten Verfahren zur Markierung und/oder Identifizierung von Biomolekülen, wie Proteinen, Peptiden, Antikörpern und Nukleinsäuren sind oftmals mit einer Vielzahl von Nachteilen verbunden:
Oftmals kommen sehr kostspielige Chemikalien zum Einsatz, welche die eigentliche Markierungs- bzw. Identifizierungsreaktion bewirken.

Des weiteren sind diese Verfahren oftmals aufwendig durchzuführen, insbesondere im Hinblick auf die Vielzahl und/oder Komplexität der durchzuführenden Verfahrensschritte.

Zudem weist die Markierung von Biomolekülen mittels Fluoreszenzfarbstoffen des Standes der Technik oftmals den gravierenden Nachteil von Aufarbeitungsverlusten durch chromatographische Separationsschritte nach der Markierungsreaktion auf, da überschüssige Farbstoffmoleküle aus dem Ansatz entfernt werden müssen.

Weiterhin liegen oftmals unspezifische Wechselwirkungen zwischen Farbstoffmolekülen des Standes der Technik, welche mitunter große Molekulargewichte aufweisen, wie den sogenannten Tandem-Dyes, und dem zu markierenden Biomolekül bzw. der zu markierenden Zelle vor, was zu Verfälschungen des Ergebnisses führen kann.

Darüber hinaus kann es im Stand der Technik zu einer Maskierung des spezifischen Fluoreszenzsignals des markierten Biomoleküls durch unspezifische Hintergrundfluoreszenz kommen, da oftmals vergleichbare bzw. ähnliche optische Eigenschaften vorliegen.

Fluoreszenzfarbstoffe des Standes der Technik weisen zudem den Nachteil auf, daß mitunter nur eine geringe Separation bzw. Differenzierung zwischen dem Anregungs- und dem Emissionsmaximum - im allgemeinen auch als Stokes-Shift bezeichnet - vorliegt, so daß das spezifische Fluoreszenzsignal oftmals nicht hinreichend von dem Anregungssignal unterschieden und ausgewertet werden kann.

Im Stand der Technik besteht ein Ansatz zur besseren Separation bzw. Differenzierbarkeit zwischen Anregungsmaximum einerseits und Emissionsmaximum andererseits darin, sogenannte Tandem-Dyes einzusetzen. Hierbei handelt es sich um Konstrukte mit zwei Fluoreszenzfarbstoffen, die über einen Fluoreszenz-Resonanz-Energietransfer von Donor zu Akzeptor eine Aufweitung des Stokes-Shift ermöglichen. Bei den Tandem-Dyes handelt es sich jedoch im allgemein um große Moleküle, was bei Interaktion mit dem zu markierenden Biomolekül oftmals mit sterischen Behinderungen einhergeht. Zudem ist die Herstellung derartiger Farbstoffe vergleichsweise kostenintensiv und aufwendig.

Weitere Nachteile organischer Fluoreszenzfarbstoffe des Standes der Technik sind darin zu sehen, daß oftmals eine sogenannte Selbstabsorption von Photonen vorliegt, was auch als "Self-Quenching" bezeichnet wird, und daß breite Emissionslinien vorliegen, welche in einigen Fällen die korrekte Detektion der Farbstoffsignale erschweren.

Die Publikation gemäß Knopf et al., "Preparation of europium-streptavidin in a time-resolved fluoroimmunoassay for interleukin-3", Journal of Immunological Methods, 1991, 138, Seiten 233 bis 236, betrifft die Herstellung von Europium-Streptavidin-Konjugaten, welche im Rahmen eines zeitaufgelösten Fluorimmunoassays als Detektionsreagenz verwendet werden.

Weiterhin betrifft die Publikation gemäß Yuan et al., "A new tetradentate β-diketonate-europium chelate that can be covalently bound to proteins for time-resolved fluoroimmunoassay", Anal. Chem., 1998, 70, Seiten 596 bis 601, die Herstellung von tetradentaten β-Diketonat-Europium-Chelaten auf Basis von chlorsulfonylierten Liganden in Form von BHHCT, welche an Proteine gebunden werden sollen.

Zudem betrifft die Publikation gemäß Yuan et al., "Lanthanoide complexbased fluorescence label for time-resolved fluorescence bioassay", J. Fluorescence, 2005, 15, Seiten 559 bis 568, Chelatkomplexe zur kovalenten Markierung von Biomolekülen unter Verwendung von BHHCT-Eu³⁺-Komplexen.

Darüber hinaus betrifft die Publikation gemäß Lim et al., "A luminescent europium complex for the sensitive detection of proteins and nucleic acids immobilized on membrane supports", Analytical Biochemistry, 1997, 245, Seiten 184 bis 195, ein Detektionsverfahren für Proteine und Nukleinsäuren, welche auf einem Membranträger fixiert bzw. immobilisiert sind.

Zudem betrifft die WO 00/39335 A1 ein Verfahren zur Durchführung einer Multiplexbestimmung auf der Oberfläche einer festen Struktur, welche unabhängig voneinander adressierbare Stellen aufweist, wobei im Rahmen des beschriebenen Verfahrens eine Mischung von Lanthanoidfarbstoffen eingesetzt und entsprechende Emissionsspektren aufgenommen werden.

Die Publikation gemäß Wu et al., "Synthesis of a highly fluorescent β-diketone-europium chelate and its utility in time-resolved fluoroimmunoassay of serum total thyroxine", Anal. Chem., 2002, 22, Seiten 5882 bis 5889, betrifft die Herstellung eines hochfluoreszierenden β-Diketon-EuropiumChelats in Form von Europium/BTOT-Chelaten und seine Verwendung in einem zeitaufgelösten Fluorimmunoassay zur Bestimmung von Thyroxin.

Weiterhin betrifft die Publikation gemäß Yao et al., "A fluorescence quenching immunoassay method for determination of trace albumin using unlabeled europium chelate", Chin. Chem. Lett., 1992, 3, Seiten 1011 bis 1014, ein Immunoessayverfahren auf Basis eines Fluoreszenzquenching von Albumin unter Verwendung von Eu-TTA-TOPO-Chelaten.

Darüber hinaus, betrifft die Publikation gemäß Castellano et al. "Longlifetime Ru(II) complexes as labeling reagents for sulfhydryl groups", Anal. Biochem., 1998, 255, Seiten 165 bis 170, eine Markierung von Proteinen, Albumin bzw. IgG unter Verwendung der Chelatkomplexe [Ru(bpy)₂(5-iodacetamido-1,10-phenanthrolin)](PF₆)₂ und Ru(bpy)₂(5-chloracetamido-1,10-phenanthrolin)](PF₆)₂.

Die Publikation gemäß Tsaryuk et al., "Effects of ligand radicals on vibrational IR, Raman and vibronic spectra of europium β-diketonates", Spectrochimica Acta Part A: Molecular and biomolecular spectroscopy, 2005, 61, Seiten 185 bis 191, betrifft die Untersuchung des Effekts von radikalischen Eigenschaften des Liganden auf die Spektren sowie die Manifestation der gegenseitigen Beeinflussung von nicht equivalenten Liganden in den Spektren von Europium β-Diketonatkomplexen.

Darüber hinaus betrifft die Publikation gemäß Peng et al., "Synthesis, characterization, and luminescent properties of the ternary europium complex covalently bonded to mesoporous SBA-15", J. Phys. Chem. B, 2005, 109, Seiten 15278 bis 15287, die Synthese, Charakterisierung und Lumineszenzeigenschaften eines ternären Europiumkomplexes, welcher kovalent an mesoporösem SBA-15 gebunden ist.

Zudem betrifft die Publikation gemäß Shen et al., "A versatile preparative route to 5-substituted-1,10-phenanthroline ligands via 1,10-phenanthroline 5,6-epoxide", Inorg. Chem., 1995, 34, Seiten 6235 bis 6236, die Synthese verschiedener 1,10-Phenthrolinliganden, welche durch Reaktion von 1,10-Phenanthrolin-5,6-epoxid mit unterschiedlichen Liganden erhalten werden können.

Weiterhin betrifft die Publikation gemäß Wilkinson D., "A one-step fluorescent detection method for lipid fingerprints; Eu(TTA)3•2TOPO", Forensic Science International, 1999, 99, Seiten 5 bis 23, die Entwicklung eines fluoreszenten Reagenzes, welches Europium-Chelate mit der allgemeinen Formel [Europium(organischer Ligand)₃•2(Synergiereagenz)] enthält, für die Detektion von Fetten.

Schließlich betrifft die Publikation gemäß Anderson et al., "Europium chelate and fluorescent brightener staining of soil propagules and their photomicrographic counting - I. Methods", Soil Biol. Biochem., 1975, 7, Seiten 205 bis 209, ein Verfahren zur direkten visuellen oder photomikrographischen Zählung von Erdkörnern in Erd-Agarfilmen und Erdabstrichen durch Färbung mittels eines Europium-Chelats in Form von Eu(TTA)₃ in Verbindung mit einem Fluoreszenzaufheller.

Daher besteht ein Bedürfnis nach einem effizient und möglichst einfach durchzuführenden Verfahren zur Markierung und/oder Identifizierung von Biomolekülen, insbesondere Peptiden, Proteinen, Antikörpern und Nukleinsäuren.

Gegenstand der vorliegenden Erfindung gemäß einem ersten Aspekt ist somit ein Verfahren zur Markierung und/oder Identifizierung mindestens eines Biomoleküls, insbesondere eines Proteins, eines Peptids, eines Antikörpers oder einer Nukleinsäure, gemäß Anspruch 1; weitere, vorteilhafte Ausgestaltungen des erfindungsgemäßen Verfahrens sind Gegenstand der diesbezüglichen Unteransprüche.

Die vorliegende Erfindung betrifft somit gemäß dem ersten Aspekt der vorliegenden Erfindung ein Verfahren zur Markierung und/oder Identifizierung mindestens eines Biomoleküls, insbesondere eines Proteins, Peptids, eines Antikörpers oder einer Nukleinsäure, wobei das Biomolekül mit mindestens einem Chelatkomplex mindestens eines Elements der Seltenen Erden ("Seltenerdkomplex") in Kontakt gebracht und hiermit unter Ausbildung einer Bindung, insbesondere koordinativen und/oder kovalenten Bindung, vorzugsweise koordinativen Bindung, zwischen Biomolekül einerseits und Chelatkomplex des Elements der Seltenen Erden andererseits zur Wechselwirkung, insbesondere zur Reaktion, gebracht wird.

Weiterer Gegenstand der vorliegenden Erfindung gemäß einem zweiten Aspekt ist die Verwendung mindestens eines Chelatkomplexes mindestens eines Elements der Seltenen Erden gemäß Anspruch 12.

Die vorliegende Erfindung betrifft somit gemäß dem zweiten Aspekt der vorliegenden Erfindung die Verwendung mindestens eines Chelatkomplexes mindestens eines Elements der Seltenen Erden zur Markierung und/oder Identifizierung mindestens eines Biomoleküls, insbesondere eines Proteins, Peptids, eines Antikörpers oder einer Nukleinsäure.

Wiederum weiterer Gegenstand der vorliegenden Erfindung gemäß einem dritten Aspekt ist die Verwendung mindestens eines Chelatkomplexes mindestens eines Elements der Seltenen Erden gemäß Anspruch 13.

Die vorliegende Erfindung betrifft somit gemäß dem dritten Erfindungsaspekt auch die Verwendung mindestens eines Chelatkomplexes mindestens eines Elements der Seltenen Erden zur Fluoreszenzmarkierung oder -identifizierung mindestens eines Biomoleküls, eines Proteins, Peptids, eines Antikörpers oder einer Nukleinsäure.

Weiterhin betrifft die vorliegende Erfindung gemäß einem vierten Aspekt ein Kit zur Markierung und/oder Identifizierung mindestens eines Biomoleküls gemäß Anspruch 14.

Schließlich betrifft die vorliegende Erfindung gemäß einem fünften Aspekt ein Biomolekül/Seltenerdkomplex-Konjugat, vorzugsweise ein Biomolekül/Lanthanoidkomplex-Konjugat, gemäß Anspruch 15.

Nachfolgend wird die Erfindung im Detail erläutert, insbesondere auch auf der Basis von verschiedenen Ausführungsbeispielen. Es versteht sich von selbst, daß Ausführungen, welche zu einem Aspekt der vorliegenden Erfindung gemacht werden, entsprechend auch für die übrigen Erfindungsaspekte gelten, ohne daß dies einer ausdrücklichen Erwähnung bedarf.

Die Anmelderin hat nun in völlig überraschender Weise herausgefunden, daß die zuvor geschilderten Nachteile des Standes der Technik durch Bereitstellung des erfindungsgemäßen Verfahrens zur Markierung bzw. Identifizierung mindestens eines Biomoleküls, insbesondere eines Proteins, eines Peptids, eines Antikörpers oder einer Nukleinsäure, gelöst werden können. Das erfindungsgemäße Verfahren zeichnet sich dadurch aus, daß das Biomolekül mit mindestens einem Chelatkomplex mindestens eines Elements der Seltenen Erden ("Seltenerdkomplex") in Kontakt gebracht und hiermit unter Ausbildung einer Bindung zwischen Biomolekül einerseits und Chelatkomplex des Elements der Seltenen Erden andererseits zur Wechselwirkung gebracht wird, wobei das Inkontaktbringen und/oder die Umsetzung von Biomolekül einerseits und Chelatkomplex des Elements der Seltenen Erden andererseits gemäß der nachfolgenden Reaktionsgleichung erfolgt: 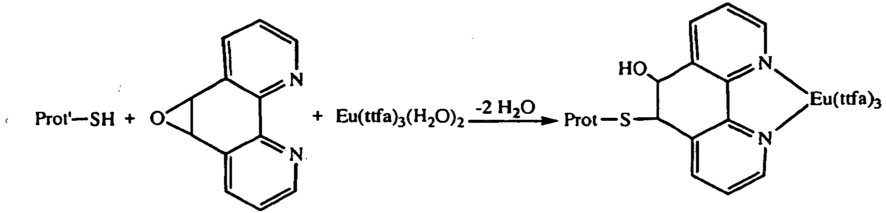 wobei:
- "Prot' " einen Biomolekülabschnitt bezeichnet,
- "Eu" einen Europium(III)-Kern bezeichnet und
- "ttfa" einen 1-(2-Thenyl)-4,4,4-trifluorbutan-1,3-dionato-Liganden bezeichnet und/oder "ttfa" einen Rest der Formel (IIa) bezeichnet:

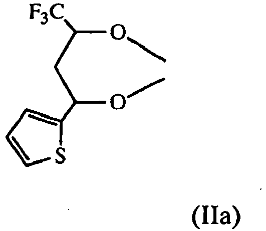

Das erfindungsgemäße Verfahren weist den entscheidenden Vorteil auf, daß die erfindungsgemäß eingesetzten Seltenerdkomplexe sich als fluoreszente Label bzw. Marker für Biomoleküle von den sonstigen organischen Fluoreszenzfarbstoffen des Standes der Technik durch ihre sehr schmalen Emissionsbanden und den langen Fluoreszenzlebenszeiten der Farbstoffsignale unterscheiden. So ist die Fluoreszenzlebenszeit der erfindungsgemäß eingesetzten Seltenerdkomplexe signifikant länger als die Hintergrundfluoreszenz organischer Verbindungen. Aufgrund der langen Fluoreszenzlebensdauern wird eine zeitliche Diskriminierung der Signale des Seltenerdkomplexes mittels zeitaufgelöster Fluoreszenzmessung ermöglicht.

Ein weiterer Vorteil des erfindungsgemäßen Verfahrens ist darin zu sehen, daß aufgrund der geringen Molekülgröße bzw. des geringen Molekulargewichtes der erfindungsgemäß eingesetzten Seltenerdkomplexe sterische Hinderungen in bezug auf das zu markierende Biomolekül nicht zu erwarten sind, so daß insgesamt eine optimale Wechselwirkung zwischen Biomolekül einerseits und Seltenerdkomplex andererseits ohne signifikante Beeinflussung der nativen Funktion des zu markierenden Biomoleküls vorliegt.

Weiterhin hat die Anmelderin in völlig überraschender Weise herausgefunden, daß sich die erfindungsgemäß eingesetzten Seltenerdkomplexe in hervorragender Weise zur Markierung von in wäßriger Lösung befindlichen Biomolekülen eignen - und dies obwohl die erfindungsgemäß eingesetzten Seltenerdkomplexe als solche im allgemeinen eine relativ geringe Wasserlöslichkeit aufweisen. Insbesondere aus diesem Grund wurde ein Einsatz der erfindungsgemäß verwendeten Seltenerdkomplexe im Bereich der biochemischen Analytik in bezug auf wäßrige Systeme bislang nicht in Betracht gezogen.

Durch das erfindungsgemäße Verfahren wird eine praktisch verlustfreie Markierung von Biomolekülen ermöglicht, da eine chromatographische Separation bzw. Abtrennung des überschüssigen Farbstoffs nicht erforderlich ist, da nur äußerst geringe bzw. vernachlässigbare Mengen des freien bzw. nichtgebundenen Seltenerdkomplexes in der Lösung verbleiben.

Zudem beschränkt sich die Interaktion des Seltenerdenkomplexes aufgrund seiner zuvor angesprochenen geringen Größe nur auf die Bindung mit dem Biomolekül als solche, und die Fluoreszenzlebensdauer übersteigt die Lebensdauer der langlebigsten bekannten organischen Komponenten um den Faktor 10, was, wie zuvor angeführt, der zeitlichen Diskriminierung des Emissionssignals zugute kommt.

Das erfindungsgemäße Verfahren ist zudem insgesamt kostengünstig und umfaßt nur wenige Verfahrensschritte und führt zu einer hervorragenden quantitativen sowie qualitativen Bestimmung von Biomolekülen der zuvor genannten Art in einer Probe.

Gegenstand der vorliegenden Erfindung - gemäß einem **ersten** Aspekt der vorliegenden Erfindung - ist somit ein Verfahren zur Markierung und/oder Identifizierung mindestens eines Biomoleküls, wobei das erfindungsgemäße Verfahren dadurch gekennzeichnet ist, daß das Biomolekül mit mindestens einem Chelatkomplex mindestens eines Elements der Seltenen Erden ("Seltenerdkomplex") in Kontakt gebracht und hiermit unter Ausbildung einer Bindung zwischen Biomolekül einerseits und Chelatkomplex des Elements der Seltenen Erden andererseits, wobei das Inkontaktbringen und/oder die Umsetzung von Biomolekül einerseits und Chelatkomplex des Elements der Seltenen Erden andererseits gemäß der nachfolgenden Reaktionsgleichung erfolgt: 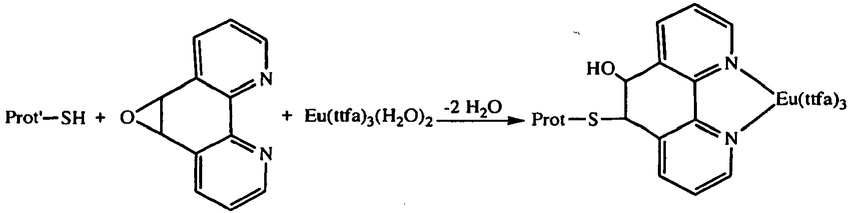 wobei:
- "Prot' " einen Biomolekülabschnitt bezeichnet,
- "Eu" einen Europium(III)-Kern bezeichnet und
- "ttfa" einen 1-(2-Thenyl)-4,4,4-trifluorbutan-1,3-dionato-Liganden bezeichnet und/oder "ttfa" einen Rest der Formel (IIa) bezeichnet:

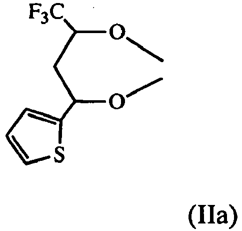

Das grundlegende Prinzip der vorliegenden Erfindung ist somit darin zu sehen, daß im Rahmen des erfindungsgemäßen Verfahrens ein spezieller Komplex, insbesondere Chelatkomplex, verwendet wird, welcher mindestens einen Kern bzw. ein Element der Seltenen Erden aufweist. Der gezielte Einsatz von Elementen der Seltenen Erden in bezug auf den zur Markierung verwendeten Komplex führt - insbesondere in Verbindung mit den erfindungsgemäß eingesetzten Liganden des Seltenerdkomplexes - in völlig überraschender Weise zu hervorragenden Emissionseigenschaften, wie engen Emissionsbanden, langen Emissions- bzw. Lumineszenzdauern, was zu einer sehr guten Detektierbarkeit einhergehend mit einer qualitativen und quantitativen Bestimmung von Biomolekülen in einer Meßprobe führt.

Aufgrund der spezifischen Eigenschaften des verwendeten Komplexes kann insgesamt eine Vielzahl von Biomolekülen markiert bzw. identifiziert werden. So eignet sich das erfindungsgemäße Verfahren in nichtbeschränkender Weise zur Markierung bzw. Identifizierung von Biomolekülen, welche aus der Gruppe von Proteinen, Peptiden, Antikörpern und Nukleinsäuren ausgebildet sind.

Was die Wechselwirkung zwischen Biomolekül einerseits und Komplex des Elements der Seltenen Erden andererseits betrifft, so ist es erfindungsgemäß vorgesehen, daß das Biomolekül an den Chelatkomplex des Elements der Seltenen Erden über eine kovalente Bindung unter Substitution mindestens eines Liganden des Komplexes des Elements der Seltenen Erden anbindet.

Weiterhin ist es im Rahmen der vorliegenden Erfindung vorgesehen, daß die Bindung des Chelatkomplexes des Elements der Seltenen Erden über mindestens eine funktionelle Gruppe des Biomoleküls erfolgt. Bei den funktionellen Gruppen handelt es sich um Thiolgruppen des Biomoleküls.

Die Bindung des Biomoleküls an den Komplex des Elements der Seltenen Erden kann unter Ausbildung eines Konjugats aus Biomolekül und Komplex des Elements der Seltenen Erden ("Biomolekül/Seltenerdkomplex-Konjugat") erfolgen.

Besonders gute Ergebnisse hinsichtlich der zuvor beschriebenen Eigenschaften des im Rahmen des erfindungsgemäßen Verfahrens verwendeten Komplexes des Elements der Seltenen Erden werden dadurch erreicht, daß das Element ausgewählt ist aus Europium.

Im Rahmen der vorliegenden Erfindung ist es vorgesehen, daß das Element der Seltenen Erden ausgewählt ist aus den Elementen der Lanthanoide, und zwar in Form von Europium.

Die Lanthanoide sind silbrig glänzende, relativ weiche und reaktionsfreudige Metalle, die an der Luft schnell oxidieren und dabei matt werden. In Wasser zersetzen sie sich mehr oder weniger schnell unter Freisetzung von Wasserstoffgas. Bei den Lanthanoiden handelt es sich im allgemeinen um insgesamt vierzehn Elemente der sechsten Periode des Periodensystems, die als Untergruppe der dritten Nebengruppe aufgefaßt werden können. Aufgrund der ähnlichen Struktur der Valenzschale verhalten sich die Lanthanoide chemisch vergleichbar zu den Elementen der dritten Gruppe des Periodensystems, nämlich Skandium und Yttrium. Mit diesen bilden die Lanthanoide die Gruppe der Seltenen Erden.

Besonders gute Ergebnisse hinsichtlich des im Rahmen der vorliegenden Erfindung eingesetzten Komplexes des Elements der Seltenen Erden werden dadurch erhalten, daß das Element der Seltenen Erden ausgewählt ist aus Europium in Form von Europium(III).

In diesem Zusammenhang hat es sich gleichermaßen als besonders vorteilhaft erwiesen, wenn das Element der Seltenen Erden Europium in Form von Europium(III) ist.

Weiterhin ist es von Vorteil, wenn der Komplex des Elements der Seltenen Erden ("Seltenerdkomplex") mindestens einkernig ist, vorzugsweise einkernig ausgebildet ist und/oder vorzugsweise ein Element der Seltenen Erden aufweist.

Was den Komplex des Elements der Seltenen Erden weiterhin anbelangt, so weist dieser mindestens einen organischen, insbesondere aromatischen und koordinativ gebundenen Liganden auf. Weiterhin weist der Komplex des Elements der Seltenen Erden mindestens einen organischen und koordinativ gebundenen Liganden auf β-Diketonatbasis zusammen mit mindestens einem Co-Liganden auf Basis von Phenanthrolinen auf. Den Liganden kommt insbesondere eine wichtige Funktion als "Antennenmoleküle" zur Absorption von Anregungsenergie zu.

Im Rahmen der vorliegenden Erfindung ist es von besonderem Vorteil, wenn der Komplex des Elements der Seltenen Erden einen Fluorophor, insbesondere einen Farbstoff, vorzugsweise einen Fluoreszenzfarbstoff umfaßt und/oder darstellt. Dies wird im Rahmen der vorliegenden Erfindung insbesondere durch die besondere Abstimmung zwischen dem Kern auf Basis des Elements der Seltenen Erden einerseits und der spezifischen Auswahl von Liganden erreicht, so daß im Rahmen der vorliegenden Erfindung insgesamt ein äußerst leistungsfähiges System zur quantitativen und qualitativen Erfassung von Biomolekülen in einer Meßprobe bereitgestellt wird. Aufgrund des spezifischen Einsatzes eines Komplexes eines Elements der Seltenen Erden in Verbindung mit spezifischen Liganden wird insgesamt ein äußerst leistungsfähiges Verfahren nach der vorliegenden Erfindung bereitgestellt, welches sich in besonderem Maße zur analytischen Verwendung im Bereich der Biochemie bzw. Molekularbiologie eignet. Dabei kann der Komplex des Elements der Seltenen Erden gewissermaßen als Universalmarker für eine Vielzahl von Biomolekülen eingesetzt werden, so daß aufgrund der vorteilhaften Unspezifität des Komplexes des Elements der Seltenen Erden eine große Anzahl von Biomolekülen quantitativ und qualitativ erfaßt werden kann.

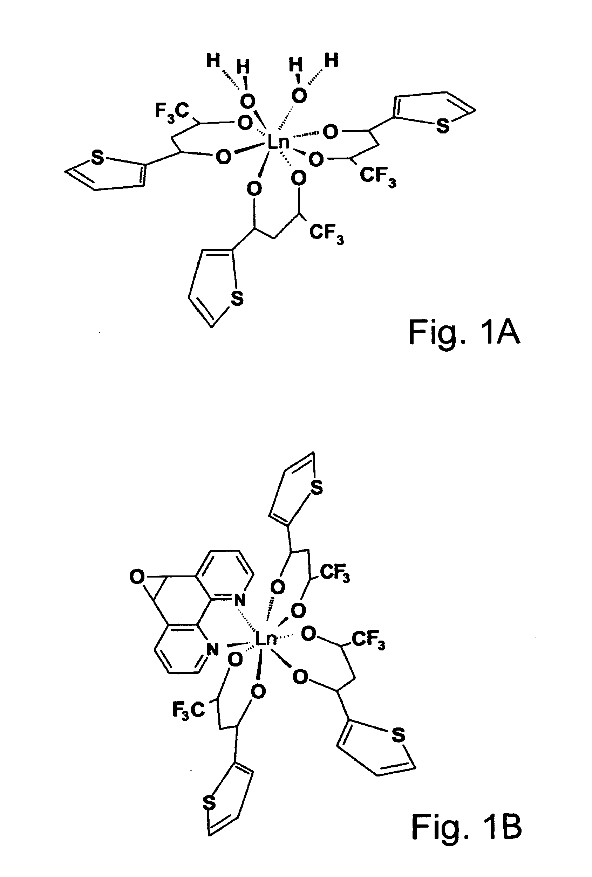

Ein Komplex des Elements der Seltenen Erden kann der Formel gemäß Fig. 1A entsprechen, wobei "Ln" ein Element der Seltenen Erden in der Form von Europium(III) darstellt (nicht erfindungsgemäß).

Erfindungsgemäß entspricht der Komplex des Elements der Seltenen Erden der Formel gemäß Fig. 1B, wobei "Ln" ein Element der Seltenen Erden in der Form von Europium(III) darstellt.

Für weitere diesbezügliche Ausführungen zu den spezifischen Komplexen des Elements der Seltenen Erden kann auf die nachfolgenden Figurenbeschreibungen zu Fig. 1A und Fig. 1B verwiesen werden.

Erfindungsgemäß entspricht der Komplex des Elements der Seltenen Erden der Formel gemäß der allgemeinen Formel (I) 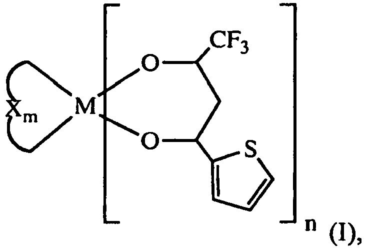 wobei:
- "M" ein Element der Seltenen Erden in der Form von Europium(III) darstellt,
- "n" die ganze Zahl 3 bezeichnet,
- "m" die ganze Zahl 1 bezeichnet und
- "X" einen Liganden der folgenden Formel darstellt:

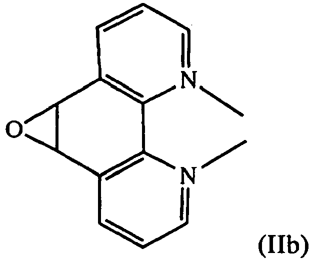

Was die im Rahmen der vorliegenden Erfindung verwendeten Komplexe des Elements der Seltenen Erden anbelangt, so sind diese als solche und deren Herstellung literaturbekannt und durch den Fachmann mit an sich geläufigen Verfahren herstellbar. Diesbezüglich kann beispielsweise verwiesen werden auf die wissenschaftliche Publikation von Shen, Y. und Sullivan, B. P., "A Versatile Preparative Route to 5-Substituted-1,10-Phenanthroline Ligands via 1,10-Phenanthroline 5,6-Epoxide", Inorg. Chem., 1995, 34, Seiten 6235 bis 6236, sowie auf die wissenschaftliche Publikation von Farah, K., Kandil, A.T., "2,2'-Dipyridyl and 1,10-Phenanthroline in the Synergetic Extraction of Eu3+", Journal of Radioanalytical and Nuclear Chemistry, Articles, Band 157, Nr. 1, 1992, Seiten 177 bis 184, und auf die wissenschaftliche Publikation von Marti, A. et al. "Structural and Photophysical Characterisation of fac-[Tricarbonyl(chloro)-(5,6-epoxy-1,10-phenanthroline)rhenium(I)]", Eur. J. Inorg. Chem. 2005, Seiten 118 bis 124, deren Offenbarungsgehalt hiermit durch Bezugnahme mitsamt den dort angeführten Literaturstellen vollumfänglich eingeschlossen ist.

Im Rahmen des erfindungsgemäßen Verfahrens kann es vorgesehen sein, daß das Reaktionsprodukt aus Biomolekül einerseits und Komplex des Elements der Seltenen Erden andererseits, insbesondere das Konjugat aus Biomolekülen und Komplex des Elements der Seltenen Erden ("Biomolekül/Seltenerdkomplex-Konjugat"), insbesondere unter Einwirkung von Anregungsenergie oder Absorption von Anregungsenergie, luminesziert, insbesondere fluoresziert. Weiterhin sollte das Reaktionsprodukt aus Biomolekül einerseits und Komplex des Elements der Seltenen Erden andererseits, insbesondere das Konjugat aus Biomolekül und Komplex des Elements der Seltenen Erden ("Biomolekül/Seltenerdkomplex-Konjugat"), insbesondere unter Einwirkung von Anregungsenergie und/oder Absorption von Anregungsenergie, detektierbare Energie, insbesondere in Form von Lumineszenz, vorzugsweise Fluoreszenz, freisetzen. In diesem Zusammenhang sollte die freigesetzte und/oder emittierte Energie von der Anregungsenergie differenzierbar und/oder unterscheidbar ausgebildet sein, vorzugsweise die Lumineszenzemissionswellenlänge von der Anregungsenergieabsorptionswellenlänge differenzierbar und/oder unterscheidbar ausgebildet sein. Zudem sollte die Lumineszenz, vorzugsweise Fluoreszenz, im Bereich des sichtbaren Lichts erfolgen. In diesem Zusammenhang sollte die Anregung mit Licht einer Wellenlänge unterhalb von 400 nm, vorzugsweise im Bereich von UV-Licht, erfolgen.

Die freigesetzte und/oder emittierte Energie kann erfindungsgemäß mittels einer Detektionsvorrichtung, insbesondere mittels eines Spektrometers, erfaßt werden, vorzugsweise qualitativ und/oder quantitativ erfaßt werden. Auf diese Weise kann gleichermaßen eine qualitative oder quantitative Erfassung des zu detektierenden Biomoleküls in der Meßprobe erfolgen. Als Detektions- bzw. Meßeinheiten kommen in nichtbeschränkender Weise auch Fluoreszenzmikroskope, Fluorimeter oder dergleichen in Betracht.

Das Inkontaktbringen und/oder die Umsetzung von Biomolekül einerseits und Komplex des Elements der Seltenen Erden andererseits sollte unter Bedingungen erfolgen derart, daß das Biomolekül, insbesondere dessen Struktur, bei der Umsetzung zumindest im wesentlichen erhalten bleibt, insbesondere das Biomolekül nicht denaturiert wird. Die diesbezüglich auszuwählenden Parameter sind insbesondere vor dem Hintergrund des jeweils zu detektierenden Biomoleküls abzustimmen bzw. auszuwählen, was dem Fachmann als solchen bekannt ist, so daß es diesbezüglich keiner weiteren Ausführungen bedarf.

Weiterhin sollte das Inkontaktbringen und/oder die Umsetzung von Biomolekül einerseits und Komplex des Elements der Seltenen Erden andererseits durchgeführt werden, indem zunächst eine Lösung und/oder Dispersion, vorzugsweise eine insbesondere wäßrige Lösung, des Biomoleküls hergestellt wird und die so hergestellte Lösung und/oder Dispersion des Biomoleküls nachfolgend mit dem Komplex des Elements der Seltenen Erden in Kontakt und zur Wechselwirkung, insbesondere Reaktion, gebracht wird, vorzugsweise hiermit umgesetzt wird, insbesondere wobei der Komplex des Elements der Seltenen Erden in fester Phase vorliegt.

Erfindungsgemäß ist es von Vorteil, wenn bei dem Inkontaktbringen und/oder bei der Umsetzung von Biomolekül einerseits und Komplex des Elements der Seltenen Erden andererseits der Komplex des Elements der Seltenen Erden in fester Phase vorliegt, insbesondere als Feststoff in Masse vorliegt oder aber an ein festes Substrat, vorzugsweise an einen hydrophoben Träger, reversibel angebunden und/oder immobilisiert und/oder fixiert ist. In diesem Zusammenhang sollte der Komplex des Elements der Seltenen Erden bedarfsweise vom Substrat bei Kontakt mit dem Biomolekül freigesetzt werden. Zudem kann in diesem Zusammenhang das feste Substrat die Wandung eines Reaktionsgefäβes, ein partikelförmiger Träger, die feste Phase einer Chromatographiereinrichtung ("HPLC-Kügelchen"), oder dergleichen sein.

Das Inkontaktbringen und/oder die Umsetzung von Biomolekül einerseits und Komplex des Elements der Seltenen Erden andererseits sollte bei einem pH-Wert im Bereich von 3 bis 8, vorzugsweise 5,5 bis 7,5, erfolgen. Gleichermaßen sollte das Inkontaktbringen und/oder die Umsetzung von Biomolekül einerseits und Komplex des Elements der Seltenen Erden andererseits bei einer Temperatur im Bereich von -20 °C bis +40 °C, vorzugsweise 0 °C bis +35 °C, erfolgen. Zudem sollte der Komplex des Elements der Seltenen Erden unter Reaktionsbedingungen nur geringfügig löslich, insbesondere nur geringfügig wasserlöslich, ausgebildet sein. In diesem Zusammenhang sollte die Löslichkeit des Komplexes des Elements der Seltenen Erden geringer als die des Biomoleküls sein, vorzugsweise um den Faktor 100, insbesondere 1.000, besonders bevorzugt 10.000.

Wie zuvor angeführt, kann es erfindungsgemäß vorgesehen sein, daß der Komplex des Elements der Seltenen Erden unspezifisch in bezug auf das Biomolekül ist. Auf diese Weise kann eine große Vielzahl von Biomolekülen bestimmt werden.

Erfindungsgemäß erfolgt das Inkontaktbringen und/oder die Umsetzung von Biomolekül einerseits und Komplex des Elements der Seltenen Erden andererseits gemäß der nachfolgenden Reaktionsgleichung: 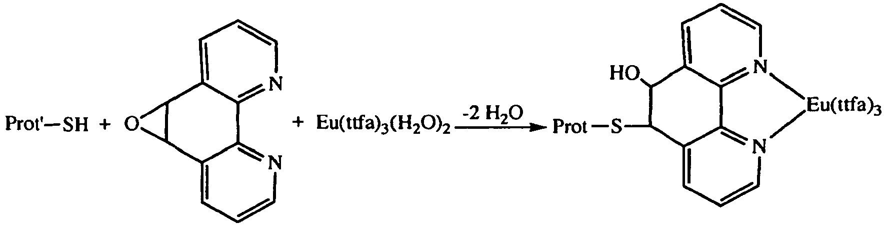 wobei:
- "Eu" einen Europium(III)-Kern bezeichnet,
- "ttfa" einen Rest der obigen Formel (IIa) bezeichnet und/oder "ttfa" einen 1-(2-Thenyl)-4,4,4-trifluorbutan-1,3-dionato-Liganden bezeichnet und
- "Prot'" einen Biomolekülabschnitt, insbesondere einen Proteinabschnitt, bezeichnet.

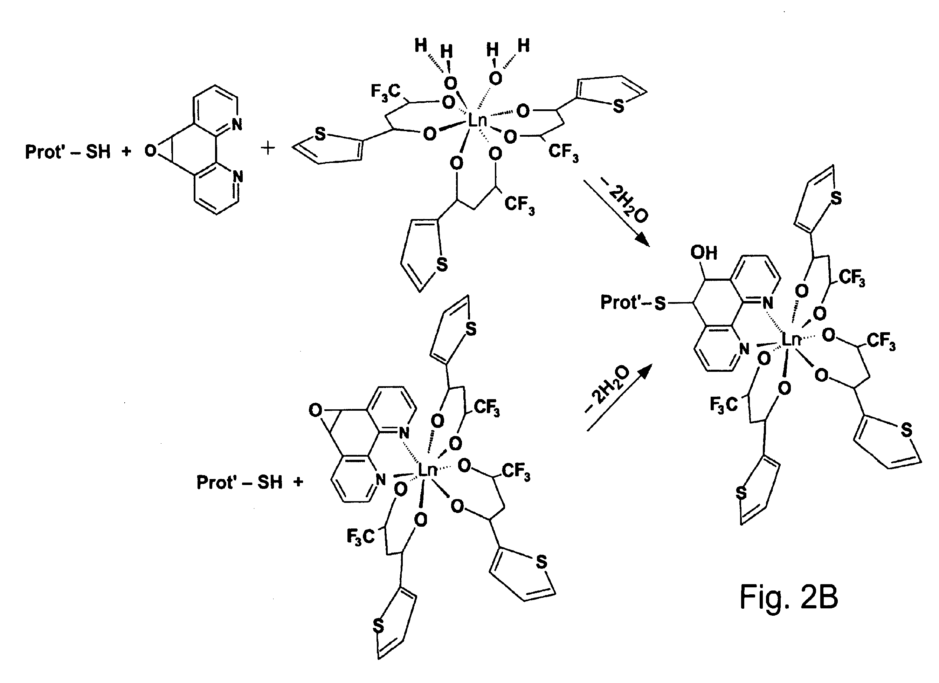

Weiterhin ist es im Rahmen der vorliegenden Erfindung vorgesehen, daß das Inkontaktbringen und/oder die Umsetzung von Biomolekül einerseits und Komplex des Elements der Seltenen Erden andererseits entsprechend der Reaktionsgleichung gemäß Fig 2B erfolgt, wobei die dort angegebenen Bezeichnungen "Prot" und "Prot' " die zuvor angegebene Bedeutung haben.

Wie zuvor angeführt, ist es im Rahmen der vorliegenden Erfindung gelungen, ein äußerst leistungsfähiges System bzw. Verfahren zur Detektion bzw. Markierung bzw. Identifizierung von Biomolekülen bereitzustellen, welches gegenüber dem Stand der Technik über signifikante Vorteile verfügt.

Weiterer Gegenstand der vorliegenden Erfindung - gemäß einem **zweiten** Aspekt der vorliegenden Erfindung - ist die Verwendung eines Chelatkomplexes mindestens eines Elements der Seltenen Erden zur Markierung und/oder Identifizierung mindestens eines Biomoleküls, insbesondere eines Proteins, eines Peptids, eines Antikörpers oder einer Nukleinsäure, wobei der Chelatkomplex des Elements der Seltenen Erden der Formel gemäß der allgemeinen Formel (I) 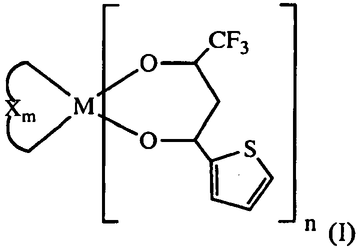 entspricht, wobei:
- "M" ein Element der Seltenen Erden in der Form von Europium(III) darstellt,
- "n" die ganze Zahl 3 bezeichnet,
- "m" die ganze Zahl 1 bezeichnet und
- "X" einen Liganden der folgenden Formel darstellt:

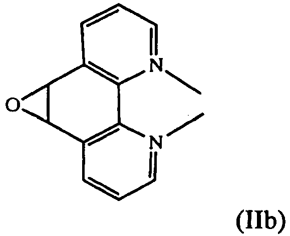

Weiterhin betrifft die vorliegende Erfindung - gemäß einem **dritten** Aspekt der vorliegenden Erfindung - die Verwendung mindestens eines Chelatkomplexes mindestens eines Elements der Seltenen Erden zur Fluoreszenzmarkierung oder -identifizierung mindestens eines Biomoleküls, insbesondere eines Proteins, eines Peptids, eines Antikörpers oder einer Nukleinsäure, wobei der Chelatkomplex des Elements der Seltenen Erden der Formel gemäß der allgemeinen Formel (I) 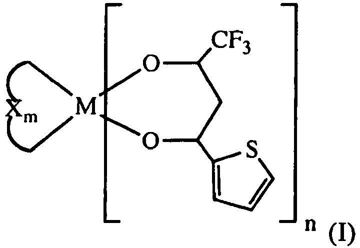 entspricht, wobei:
- "M" ein Element der Seltenen Erden in der Form von Europium(III) darstellt,
- "n" die ganze Zahl 3 bezeichnet,
- "m" die ganze Zahl 1 bezeichnet und
- "X" einen Liganden der folgenden Formel darstellt:

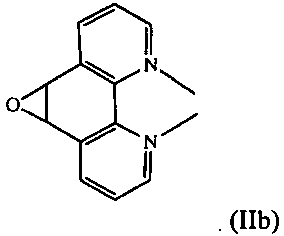

In bezug auf den im Rahmen der erfindungsgemäßen Verwendungen gemäß dem zweiten und dritten Aspekt der vorliegenden Erfindung eingesetzten Komplex des Elements der Seltenen Erden sowie in bezug auf weitere Ausführungen kann auf die obigen Ausführungen zu dem ersten Aspekt der vorliegenden Erfindung verwiesen werden, welche für die Verwendungen nach der Erfindung entsprechend gelten.

Weiterhin betrifft die vorliegende Erfindung - gemäß einem **vierten** Aspekt der vorliegenden Erfindung - ein Kit zur Markierung und/oder Identifizierung mindestens eines Biomoleküls, umfassend mindestens ein Reaktionsgefäß und/oder einen abgegrenzten Reaktionsraum, wobei in dem Reaktionsgefäß und/oder Reaktionsraum mindestens ein Chelatkomplex mindestens eines Elements der Seltenen Erden ("Seltenerdkomplex") befindlich ist, wobei der Chelatkomplex des Elements der Seltenen Erden der Formel gemäß der allgemeinen Formel (I) 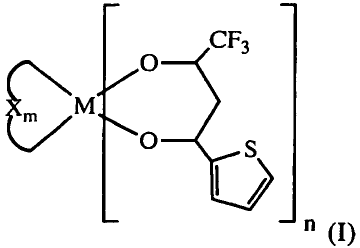 entspricht, wobei:
- "M" ein Element der Seltenen Erden in der Form von Europium(III) darstellt,
- "n" die ganze Zahl 3 bezeichnet,
- "m" die ganze Zahl 1 bezeichnet und
- "X" einen Liganden der folgenden Formel darstellt:

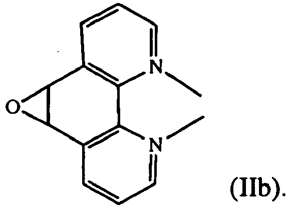

Insbesondere kann der Komplex des Elements der Seltenen Erden in fester Phase vorliegen, insbesondere als Feststoff in Masse vorliegen oder aber an ein festes Substrat, vorzugsweise an einen hydrophoben Träger, reversibel angebunden und/oder immobilisiert und/oder fixiert sein, insbesondere wobei der Komplex des Elements der Seltenen Erden bedarfsweise vom Substrat bei Kontakt mit dem Biomolekül freisetzbar ausgebildet ist und/oder insbesondere wobei das feste Substrat die Wandung des Reaktionsgefäßes und/oder Reaktionsraums, insbesondere dessen Verschlußeinrichtung, ein partikelförmiger Träger, die feste Phase einer Chromatographiereinrichtung ("HPLC-Kügelchen") oder dergleichen ist.

Schließlich betrifft die vorliegende Erfindung - gemäß einem **fünften** Aspekt der vorliegenden Erfindung - ein Biomolekül/Seltenerdkomplex-Konjugat, insbesondere Biomolekül/Lanthanoidkomplex-Konjugat, erhältlich durch Inkontaktbringen und/oder Umsetzung, insbesondere Reaktion mindestens eines Biomoleküls einerseits und mindestens eines Chelatkomplexes mindestens eines Elements der Seltenen Erden ("Seltenerdkomplex") andererseits, wobei der Chelatkomplex des Elements der Seltenen Erden der Formel gemäß der allgemeinen Formel (I) 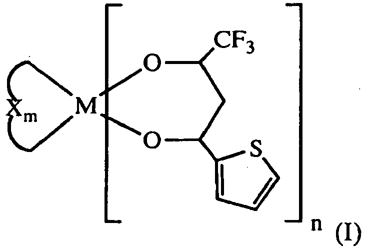 entspricht, wobei:
- "M" ein Element der Seltenen Erden in der Form von Europium(III) darstellt,
- "n" die ganze Zahl 3 bezeichnet,
- "m" die ganze Zahl 1 bezeichnet und
- "X" einen Liganden der folgenden Formel darstellt:

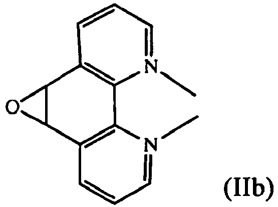

Für weitere Ausführungen betreffend den vierten und fünften Aspekt der vorliegenden Erfindung kann auf die obigen Ausführungen zu dem ersten bis dritten Aspekt der vorliegenden Erfindung verwiesen werden, welche entsprechend gelten.

Die vorliegende Erfindung wird anhand der Figurendarstellungen rein exemplarisch weiter veranschaulicht, ohne die Erfindung jedoch hierauf zu beschränken:

Fig. 1A zeigt einen verwendeten Lanthanoidkomplex, wobei es sich hierbei um Ln(ttfa)₃(H₂O)₂ bzw. [Tris(1-(2-thenyl)-4,4,4-trifluorbutan-1,3-dionato)-(diaquo)]-Ln handelt, wobei Ln durch ein Lanthanoid, insbesondere durch Europium, vorzugsweise Eu(III), gebildet ist und "ttfa" den zuvor genannten 1-(2-Thenyl)-4,4,4-trifluorbutan-1,3-dionato-Liganden bezeichnet (nicht erfindungsgemäß). Der Lanthanoidkomplex gemäß Fig. 1A eignet sich insbesondere zur Markierung bzw. Identifizierung von Biomolekülen, vorzugsweise mittels Interaktion bzw. Ausbildung einer vorzugsweise koordinativen Bindung, z. B mit einer Carboxylgruppe des Biomoleküls (z. B. einem Protein = "Prot" bzw. Prot' "), wobei im Rahmen der zugrundeliegenden Reaktion ein Molekül ttfa-Ligand sowie zwei Wassermoleküle von je einem Molekül Lantanoidkomplex abgespalten werden können.

Fig. 1B zeigt einen im Rahmen der vorliegenden Erfindung verwendeten erfindungsgemäßen Lanthanoidkomplex, wobei es sich hierbei um Ln(ttfa)₃(epoxyphen) bzw. [(5,6-Epoxy-1,10-phenanthrolino)-tris(1-(2-thenyl)-4,4,4-triflorbutan-1,3-dionato)]-Ln handelt, wobei Ln durch Eu(III) gebildet ist, der Ligand "ttfa" die zuvor angegebene Bezeichnung aufweist und der Ligand "epoxyphen" den in Fig. 1B dargestellten 5,6-Epoxy-1,10-phenanthrolino-Liganden bezeichnet. Der Lanthanoidkomplex gemäß Fig. 1B eignet sich insbesondere zur Markierung bzw. Identifizierung von Biomolekülen, welche Thiolgruppen (SH-Gruppen) aufweisen, wobei die Interaktion mit der Thiolgruppe des Biomoleküls bzw. die Ausbildung einer Bindung mit der Thiolgruppe des Biomoleküls über die Epoxygruppe des 5,6-Epoxy-1,10-phenanthrolino-Liganden des Lanthanoidkomplexes resultiert.

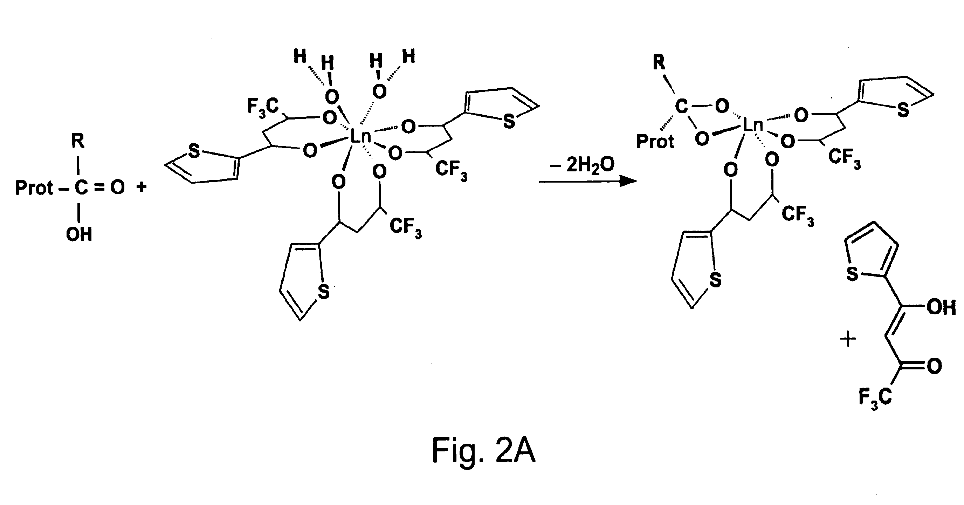

Fig. 2A verdeutlicht den der Markierung bzw. Identifizierung von Biomolekülen zugrundeliegenden Reaktionsablauf unter Verwendung des Komplexes Ln(ttfa)₃(H₂O)₂ gemäß Fig. 1A als Lanthanoidkomplex, wobei als Reaktionsprodukt ein Biomolekül/Lanthanoidkomplex-Konjugat resultiert. Gemäß Fig. 2A bedeutet die Abkürzung "Prot" das zu markierende Biomolekül, insbesondere Protein. Bei der Markierungsreaktion resultiert eine vorzugsweise koordinative Bindung des Lanthanoidkomplexes einerseits mit dem Biomolekül andererseits unter Abspaltung von einem ttfa-Liganden und Freisetzung von zwei Wassermolekülen pro Molekül Lanthanoidkomplex. Der Lanthanoidkomplex interagiert dabei z. B. mit einer Carboxylgruppe des Biomoleküls.

Fig. 2B veranschaulicht einen weiteren Reaktionsmechanismus, welcher dem erfindungsgemäßen Verfahren zur Markierung bzw. Identifizierung von Biomolekülen zugrundeliegt. Als Lanthanoidkomplex wird diesbezüglich der gemäß Fig. 1B dargestellte Lanthanoidkomplex eingesetzt. Es resultiert ein Biomolekül/Lanthanoidkomplex-Konjugat, wobei der Lanthanoidkomplex unter Öffnung des Epoxidrings des 5,6-Epoxy-1,10-phenanthrolino-Liganden des Lanthanoidkomplexes durch Reaktion mit einer Thiolgruppe des Biomoleküls kovalent an das Biomolekül gebunden ist. Die Bezeichnung "Prot' " gemäß Fig. 2B wird entsprechend zu der Bezeichnung "Prot" gemäß Fig. 2A verwendet.

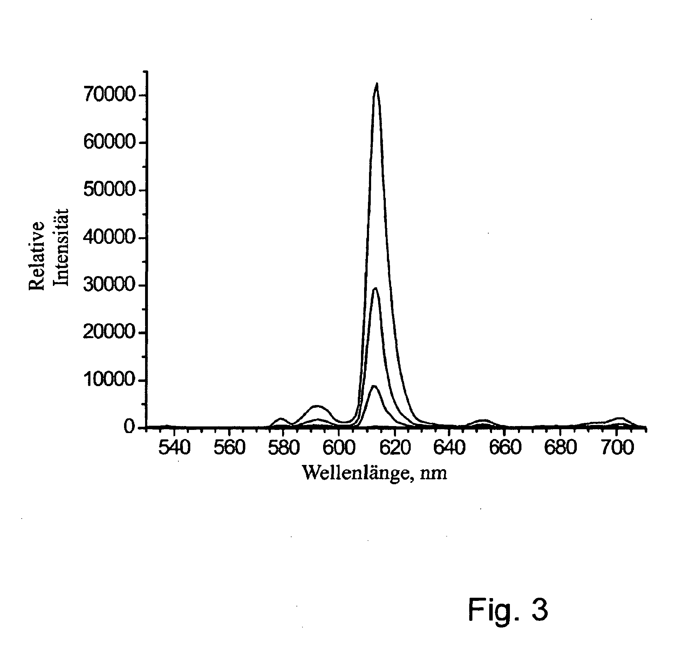

Fig. 3 zeigt das Emissionsspektrum von Biomolekül/Lantanoidkomplex-Konjugaten in Abhängigkeit von der Biomolekülkonzentration der eingesetzten Lösung (nicht erfindungsgemäß). Als Biomolekül wird z. B. Bovines Serumalbumin (BSA) eingesetzt. Als Lanthanoidkomplex wird Ln(ttfa)₃(H₂O) gemäß Fig. 1A eingesetzt, wobei als Lanthanoid Eu(III) verwendet wird. Die BSA-Konzentration in den jeweiligen Lösungen beträgt 2 x 10⁻² mg/ml (obere Kurve), 2 x 10⁻³ mg/ml (mittlere Kurve) bzw. 2 x 10⁻⁵ mg/ml (untere Kurve). Fig. 3 verdeutlicht, daß die relative Emissionsintensität mit der BSA-Konzentration in der Lösung steigt. Das erfindungsgemäße Verfahren eignet sich somit zur qualitativen wie quantitativen Bestimmung des Proteingehaltes in einer Lösung. Für sämtliche Proteinkonzentrationen resultiert ein scharfes bzw. engbandiges Emissionsmaximum im Bereich von 612 nm, was zu einer hervorragenden Detektierbarkeit der markierten Proteine führt. Neben der hohen Fluoreszenzintensität resultiert eine relativ lange, gut detektierbare Fluoreszenzdauer bzw. -lebenszeit. Die Markierung des in Form von BSA eingesetzten Proteins kann erfolgen, indem der Lanthanoidkomplex mit der Lösung des Proteins in Kontakt gebracht wird. Diesbezüglich kann beispielsweise derart verfahren werden, daß der Lanthanoidkomplex zunächst an einer vorzugsweise hydrophoben Oberfläche z. B. eines Reaktionsgefäßes reversibel fixiert bzw. gebunden wird und anschließend eine Lösung des zu markierenden bzw. zu identifizierenden Proteins in das Reaktionsgefäß gegeben wird. Nachfolgend sollte das Reaktionsgefäß z. B. geschüttelt werden, um ein zumindest teilweises Ablösen des Lanthanoidkomplexes von der Oberfläche des Reaktionsgefäßes zu ermöglichen. Ohne sich auf diese Theorie beschränken zu wollen, können die der Markierungsreaktion zugrundeliegenden Mechanismen bzw. Vorgänge darauf beruhen, daß Spuren des an sich nicht wasserlöslichen Lanthanoidkomplexes in die Lösung überführt werden und mit dem zu markierenden bzw. zu identifizierenden Protein interagieren. Dabei ist die Menge an in der Lösung befindlichen Lanthanoidkomplexen äußerst gering, da nahezu sämtliche in Lösung gelagerte Lanthanoidkomplexe sofort abreagieren, so daß eine nachträgliche Abtrennung des markierten Proteins bzw. von nichtreagiertem Lanthanoidkomplex entfallen kann. Grundsätzlich kann jedoch auch eine Aufreinigung, beispielsweise durch Filtration bzw. Zentrifugation, erfolgen. Der zugrundeliegende Reaktionsmechanismus kann dabei so verstanden werden, daß - ohne sich hierauf festlegen zu wollen - bei der Reaktion eines Moleküls des Lanthanoidkomplexes mit dem Protein je Molekül des Lanthanoidkomplexes ein Molekül Ligand (d. h. unkoordiniertes Htffa) sowie zwei Moleküle Wasser aus dem Lanthanoidkomplex freigesetzt werden und der Lanthanoidkern mit den beiden Sauerstoffatomen der Carboxylgruppe des Proteins ein Protein/Lanthanoidkomplex-Konjugat ausbildet.

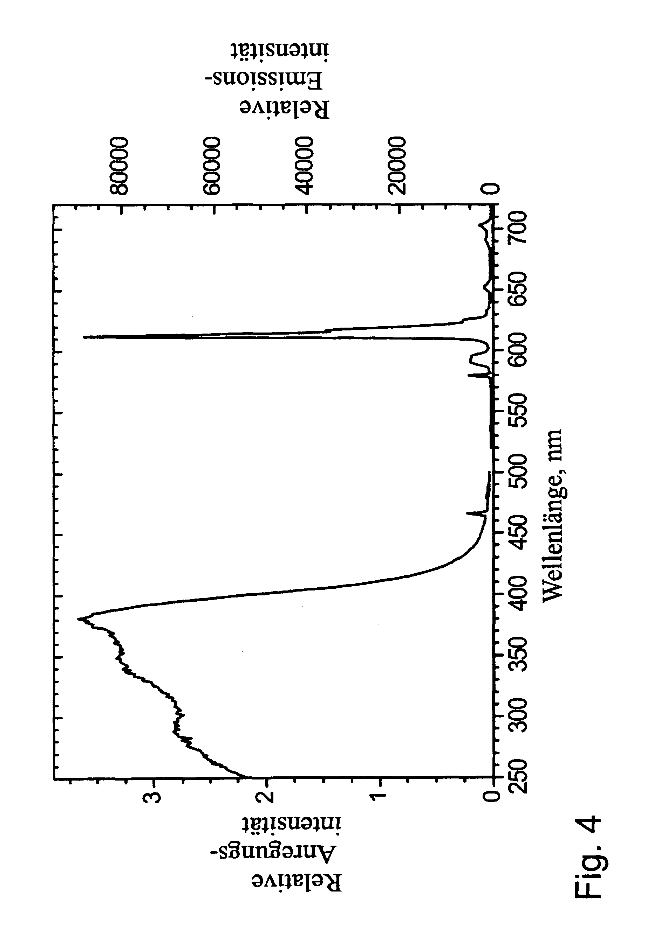

Fig. 4 zeigt das Anregungs- und Lumineszenz- bzw. Emissionsspektrum eines Biomolekül/Lanthanoidkomplex-Konjugats. Als Biomolekül wurde das Tripeptid Glutathion (GSH) eingesetzt, und als Lanthanoidkomplex wurde ein erfindungsgemäßes Eu(tffa)₃(epoxyphen) gemäß Fig. 1B eingesetzt. Es resultiert ein scharfes bzw. engbandiges Emissionsmaximum bei 612 nm. Die Zeitkonstante τ der Emission des Eu(tffa)₃(phen)/GSH-Konjugats beträgt etwa 450 µs.

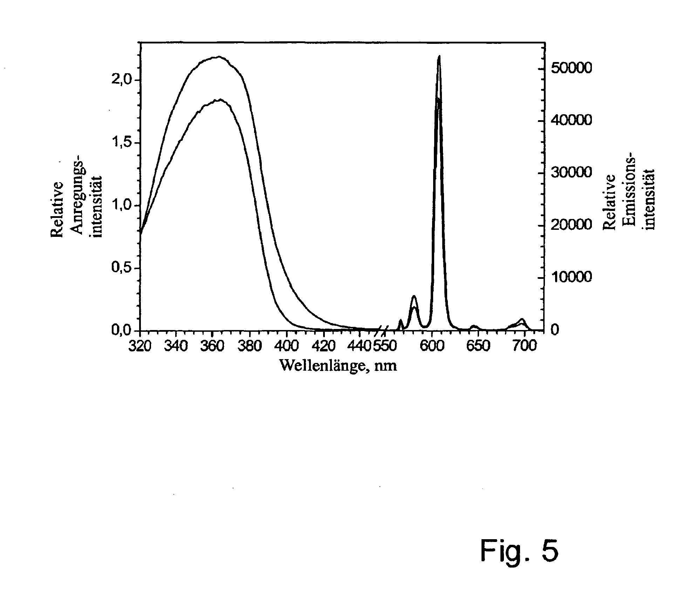

Fig. 5 veranschaulicht einen Vergleich der Anregungs- und Lumineszenz- bzw. Emissionsspektren von GSH/Eu(ttfa)₃-Konjugaten einerseits (jeweils obere Kurve des Anregungsspektrums und des Emissionsspektrums) und von Konjugaten aus BSA und Eu(ttfa)₃-Lanthanoidkomplexen andererseits (jeweils untere Kurve des Anregungsspektrums und des Emissionsspektrums), wobei im letzteren Fall ein BSA/Eu(ttfa)₂-Konjugat resultiert. Als Lanthanoidkomplex wurde in beiden Fällen ein Komplex gemäß Fig. 1A eingesetzt, wobei als Lanthanoid Eu(III) eingesetzt wurde. Die Zeitkonstante des Emissionsverhaltens beträgt für die GSH/Eu(ttfa)₃-Konjugate τ = 580 ± 2 µs. Für die BSA/Eu(ttfa)₂-Konjugate beträgt die Zeitkonstante τ = 487 ± 1 µs. Für beide Fälle resultiert ein schmales bzw. engbandiges und gut auflösbares Emissionsmaximum bei etwa 615 nm. Das erfindungsgemäße Verfahren zeigt somit sowohl für Proteine mit relativ großem Molekulargewicht, wie BSA, als auch für kleine Modellpeptide, wie GSH, hervorragende Markierungsergebnisse. Während - ohne sich hierauf beschränken zu wollen - bei der Ausbildung der BSA/Eu(ttfa)₂-Konjugate eine Abspaltung eines ttfa-Liganden ("Httfa") zu beobachten ist, erfolgt bei der Ausbildung der GSH/Eu(ttfa)₃-Konjugate keine solche Ligandenabspaltung.

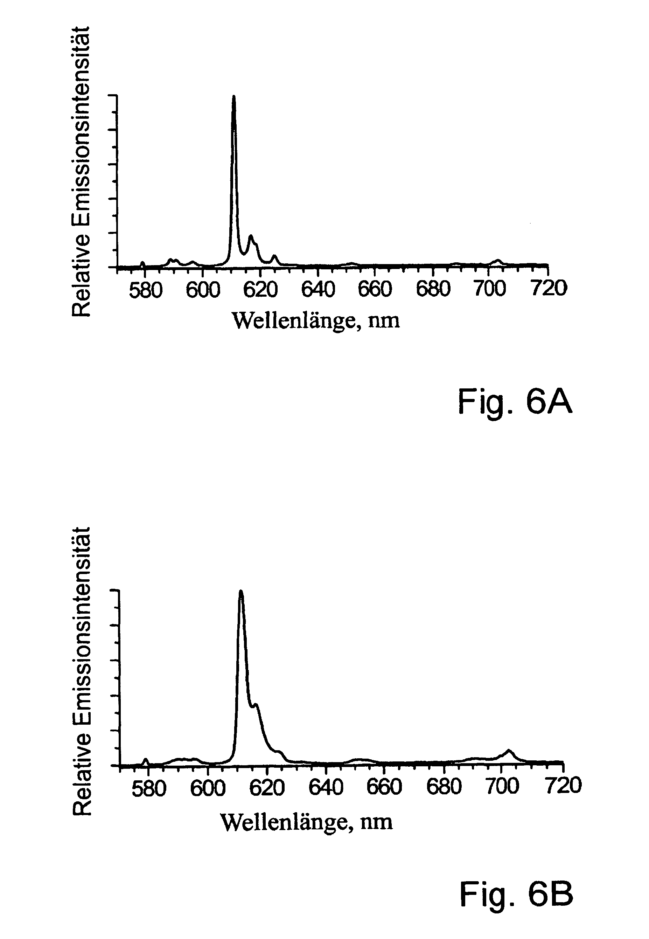

Weiterhin zeigen Fig. 6A und Fig. 6B als Vergleichsbeispiel das Emissionsverhalten eines Eu(ttfa)₃(phen)-Komplexes als solchen (mit "phen" = 1,10-Phenanthrolin) (Fig. 6A) sowie von in Polymethylmethacrylat (PMMA) eingearbeitetes bzw. eingebrachtes Eu(ttfa)₃(phen) (Fig. 6B). Bei dem eingesetzten Lanthanoidkomplex handelt es sich somit hinsichtlich des Phenanthrolin-Liganden um einen solchen Liganden, welcher keine Epoxygruppe besitzt. Der Eu(ttfa)₃(phen)-Komplex liegt in der PMMA-Matrix somit ohne Ausbildung einer Bindung mit der Matrix vor. Die Beispiele gemäß Fig. 6A und 6B fungieren als Referenzbeispiele hinsichtlich der Beurteilung der Leistungsfähigkeit der nachfolgenden erfindungsgemäßen Beispiele.

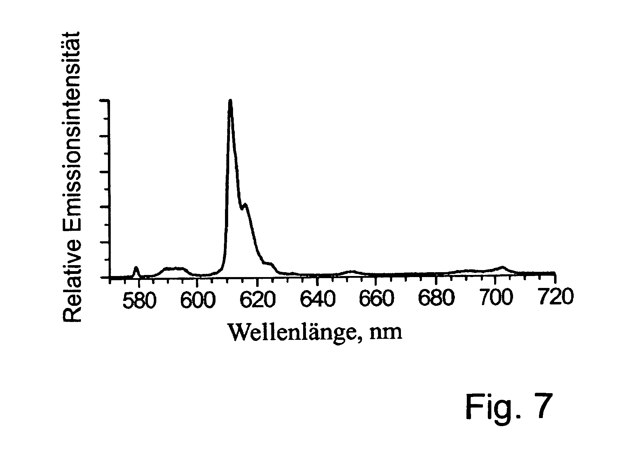

In diesem Zusammenhang zeigt Fig. 7 das Emissionsspektrum eines erfindungsgemäßen Konjugats, welches durch Interaktion von Eu(ttfa)₃(epoxyphen) mit Glutathion (GSH) erhalten wird (vgl. Fig. 4). Es resultiert eine Emissionsbande, welche klar definiert und engbandig ist. Das Emissionsverhalten des Konjugats gemäß Fig. 7 ist vergleichbar zu dem Referenzbeispiel gemäß Fig. 6B mit der Folge, daß das erfindungsgemäße Verfahren auf Basis der in Fig. 7 eingesetzten Lanthanoidkomplexe äußerst leistungsfähig ist und zu gut detektierbaren und analysierbaren Emissionsbanden führt.

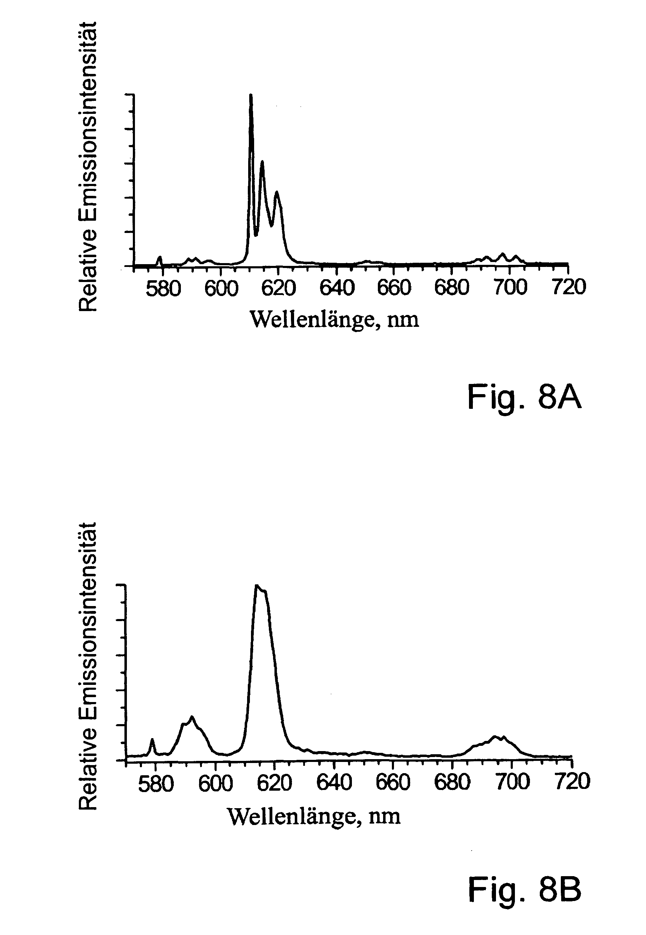

Fig. 8A zeigt das Emissionsspektrum eines Eu(ttfa)₃(H₂O)₂-Komplexes gemäß Fig. 1A als solchen, und Fig. 8B zeigt das Emissionsspektrum des Eu(ttfa)₃(H₂O)₂-Komplexes in einer PMMA-Matrix. Während für das Emissionsverhalten des Eu(ttfa)₃(H₂O)₂-Komplexes als solchen eine Zeitkonstante von τ = 198 ± 2 µs bestimmt wird, beträgt die Zeitkonstante für den Eu(ttfa)₃(H₂O)₂-Komplex in der PMMA-Matrix 362 ± 3 µs. Es resultiert somit eine Verlängerung der Emission des Komplexes bei Einbringen in eine Matrix.

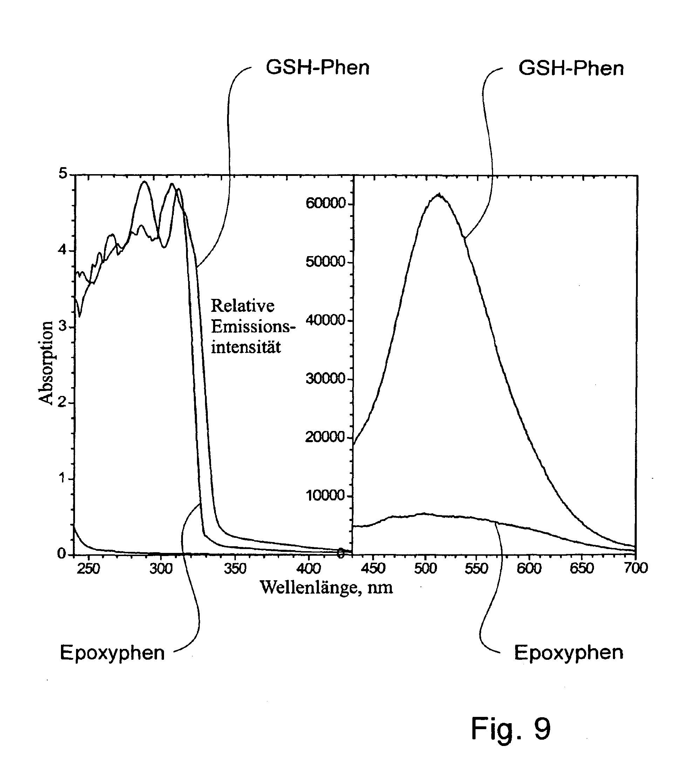

Fig. 9 zeigt das Absorptions- und Lumineszenz- bzw. Emissionsspektrum eines Komplexes, welcher durch Reaktion von Glutathion (GSH) mit Epoxyphenanthrolin (Epoxyphen) erhalten wurde ("GSH-Phen"). Das resultierende Konjugat weist somit keinen Chelatkomplex eines Elements der Seltenen Erden und somit keinen Lanthanoidkern auf. Fig. 9 zeigt weiterhin das Absorptions- und Lumineszenz- bzw. Emissionsspektrum von Epoxyphenanthrolin ("Epoxyphen") als solches und damit des erfindungsgemäß für die entsprechenden Lanthanoidkomplexe eingesetzten Liganden, jedoch ohne entsprechenden Lanthanoidkern. In beiden Fällen resultieren relativ unspezifische Emissionsverhalten ohne Ausbildung eines diskreten und engbandigen Emissionsmaximums bzw. Emissionspeaks (Vergleichsbeispiele).

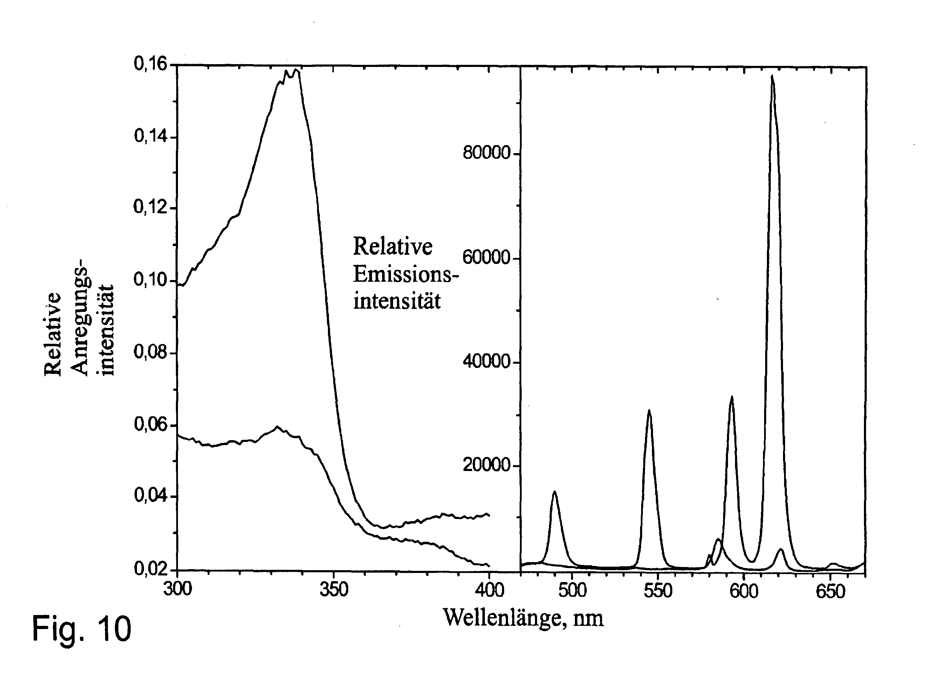

Fig. 10 zeigt schließlich das Absorptions- und Lumineszenz- bzw. Emissionsspektrum von Konjugaten, wobei als Biomolekül der Zuckeralkohol Chitosan eingesetzt wird. Als Komplex des Elements der Seltenen Erden wurde ein erfindungsgemäßer Eu(III)-Komplex, welcher einen wie zuvor beschriebenen Epoxyphenanthrolinliganden (1,5-Epoxy-1,10-Phenanthrolin) aufweist (obere Kurve bzw. Kurve mit Emissionsmaximum bei etwa 615 nm), bzw. ein Tb(III)-Komplex mit einem wie zuvor beschriebenen Epoxyphenathrolin-Liganden (untere Kurve bzw. Kurve mit Emissionspik bei etwa 545 nm) eingesetzt. Die verwendeten Komplexe der Seltenen Erden besitzen jeweils fünf koordinierte Wassermoleküle. Die jeweiligen Lanthanoide werden in Form von LnCl₃ eingesetzt. Es resultieren somit Chitosan/Ln-Phen-Konjugate mit den in Fig. 10 dargestellten Emissionsverhalten. In bezug auf das mit dem Eu(III)-Komplex markierte Chitosan resultiert ein Emissionsmaximum bei etwa 615 nm, während in bezug auf das Konjugat mit dem Tb(III)-Komplex ein Emissionsmaximum bei etwa 545 nm vorliegt.

Zusammenfassend fokussiert die vorliegende Erfindung auf ein lumineszenzoptisches Verfahren, die Verwendung spezieller Komplexe von Seltenen Erden und Materialkombinationen, auf dessen Basis eine besonders vereinfachte, empfindliche und kostengünstige Bestimmung von Biomolekülen, insbesondere wie zuvor angeführt, ermöglicht wird.

Besondere Vorteile der erfindungsgemäß eingesetzten Komplexe bestehen in ihrer Verfügbarkeit und einfachen Herstellungsmethode, ihren günstigen Lumineszenzeigenschaften (großer Stokes-Shift, lange Lebensdauern der angeregten Zustände, effiziente und breitbandige Anregung durch den sogenannten Antenneneffekt, schmalbandige Emissionslinien) und - im Rahmen der hier beschriebenen Analysenmethodik - in der genannten Wasserunlöslichkeit der Komplexe.

Die langen Lebensdauern der angeregten Zustände können genutzt werden, um eine hervorragende Differenzierbarkeit des Emissionsspektrums gegenüber den Anregungsspektrum und/oder der unspezifischen Fluoreszenz zu realisieren.

Im Rahmen von experimentellen Untersuchungen können die Feststoff Quantenausbeuten eines aus einer Lösung isolierten Protein/Seltenerdkomplex-Konjugates Eu(ttfa)₂BSA zu 14,5 % bestimmt werden; die zugehörigen Lumineszenzabklingzeiten betragen 487 µs, wohingegen bei einer Vergleichsmessung der Abklingzeit des reinen Eu(ttfa)₃(H₂O)₂ unter gleichen Meßbedingungen lediglich 196 µs ermittelt werden können.

Die Koordinationsänderung am Eu³⁺-Zentrum kann weiterhin durch einen Vergleich der Emissionsspektren des besonders effizienten Komplexes Eu(ttfa)₃phen (Abklingzeit 945 µs, phen = 1,10-Phenanthrolin) und Eu(ttfa)₃(H₂O)₂ mit Eu(ttfa)₂BSA belegt werden, indem sich symmetriebedingt das charakteristische Emissionsmuster des Eu(ttfa)₂BSA im Bereich der ⁵D₀ → ⁷F₂ Emissionen denen des Eu(ttfa)₃phen stark annähert. Die langen Lebensdauern der angeregten Zustände der BSA/Seltenerdkomplex-Konjugate können zudem zu einer Diskriminierung der Autofluoreszenz der organischen Proben ausgenutzt werden.

In diesem Zusammenhang kann auf Fig. 6A mit Eu(ttfa)₃phen, Fig. 3 mit Eu(ttfa)₂BSA und Fig. 8A mit Eu(ttfa)₃(H₂O)₂ verwiesen werden.

Das Prinzip der Markierung mittels des erfindungsgemäßen Verfahrens auf Basis der Seltenerd(chelat)komplexe fokussiert auf β-Diketonat-Liganden, die hohe Absorptionen und hohe Quantenausbeuten ermöglichen. Als etwaige weitere Liganden ("Co-Liganden"), insbesondere zur Schließung der Koordinationssphäre und/oder zur Beeinflussung des Absorptionsverhaltens, können Bipyridine und Phenanthroline sowie deren substituierte Derivate in Betracht kommen.

Die Liganden dienen einerseits sozusagen als Antennen für die Anregungsstrahlung und andererseits für die Abschirmung des zentralen Seltenerd-Ions zum Schutz vor Quenching, wie es z. B. durch Wasser verursacht werden kann.

Grundsätzlich kann aber auch auf den Einsatz von Co-Liganden verzichtet werden, so daß in bezug auf die Komplexe unvollständige Koordinationssphären vorliegen können. Die verbleibenden β-Diketonat-Liganden können dann gegebenenfalls mit dem Biomolekül interagieren, wobei - ohne sich hierauf festlegen zu wollen - einer der Liganden gegen eine funktionelle Gruppe, insbesondere eine Carboxylgruppe, des Biomoleküls ausgetauscht werden kann.

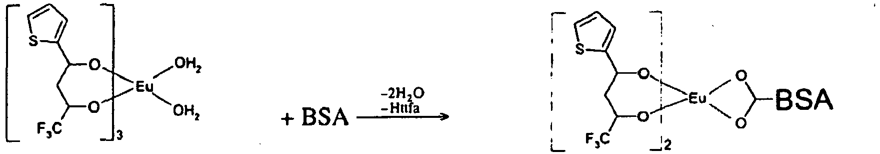

Das resultierende Protein/Seltenerdkomplex-Konjugat profitiert dabei von der geringen Wasserlöslichkeit des freigesetzten bzw. aus dem Komplex ausgetretenen Liganden "Httfa" und des Eu(ttfa)₃.

Die verbleibenden "Httfa" binden z. B. an hydrophobe Oberflächen, wie z.B. an ein hydrophobes bzw. hydrophobisiertes Reaktionsgefäß, und zum anderen ist der Komplex Eu(ttfa)₃ in Lösung fast nicht vorhanden.

Die einfache Entnahme der Suspension kann zudem bereits zu einer Separation von überschüssigen Farbstoff und Nebenprodukten führen.

Eine Markierungsprozedur auf Basis des zuvor beschriebenen Verfahrens kann beispielsweise wie folgt durchgeführt werden:
- Der Feststoff Eu(ttfa)₃ wird in einem organischen Lösungsmittel gelöst (z.B. Acetonitril oder Toluol);
- die Lösung wird in ein hydrophobes bzw. hydrophobisiertes Gefäß, z. B. ein hydrophobes bzw. hydrophobisiertes Reaktionsgefäß bzw. -behältnis, gegeben;
- in einer Vakuumzentrifuge oder durch simples Abdampfen wird das Lösungsmittel entfernt und der Feststoff gleichmäßig an der inneren Oberfläche des Reaktionsgefäßes verteilt;
- die Proteinlösung wird in das vorbereitete Gefäß gegeben und bis zu acht Stunden geschüttelt (alternativ können zur Oberflächenvergrößerung auch Eu(ttfa)₃ belegte Mikrosphären oder HPLC-Partikel zugegeben werden, was zu einer Verkürzung der Inkubationszeit führt);
- Zentrifugation, um Mikrokristalle zu entfernen; und schließlich
- Entnahme der markierten Proteinlösung.

Eigenschaften des markierten Proteins:
- Anregung im UV zwischen 300 und 390 nm;
- Emission bei 612 ± 5 nm;
- Fluoreszenzlebensdauer typischerweise 0,5 ms;

Weitere Vorteile und Eigenschaften:
- Europium-Filter sind kommerziell für viele Spektroskope erhältlich;
- optimal funktioniert die Markierung in HEPES- oder MES-Puffer;
- die Nachweisgrenze liegt mit dem erfindungsgemäßen Verfahren im gleichen Bereich wie kommerziell erhältliche Systeme, z. B. Nano Orange^{®} von Molecular Probes;
- das erfindungsgemäße Verfahren funktioniert mit Antikörpern und rohen Proteinlösungen;
- die Toxizität von Seltenen Erden ist sehr gering, Verfütterung an Ratten ergab über drei Generationen hinweg keine Schäden (Haley, T. J., "Handbook on the Physics and Chemistry of Rare Earths", ed. K.A. Gschneider Jr., L. Eyering, Chapter 40, North-Holland Publishing Company, 1979).

Weitergehende Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

## Patentansprüche

1. Verfahren zur Markierung und/oder Identifizierung mindestens eines Biomoleküls,
wobei das Biomolekül mit mindestens einem Chelatkomplex mindestens eines Elements der Seltenen Erden ("Seltenerdkomplex") in Kontakt gebracht und hiermit unter Ausbildung einer Bindung zwischen Biomolekül einerseits und Chelatkomplex des Elements der Seltenen Erden andererseits zur Wechselwirkung gebracht wird,
wobei das Inkontaktbringen und/oder die Umsetzung von Biomolekül einerseits und Chelatkomplex des Elements der Seltenen Erden andererseits gemäß der nachfolgenden Reaktionsgleichung erfolgt: 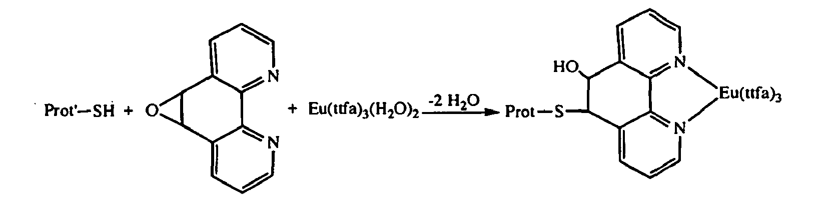 wobei:
• "Prot' " einen Biomolekülabschnitt bezeichnet,
• "Eu" einen Europium(III)-Kern bezeichnet und
• "ttfa" einen 1-(2-Thenyl)-4,4,4-trifluorbutan-1,3-dionato-Liganden bezeichnet und/oder "ttfa" einen Rest der Formel (IIa) bezeichnet:

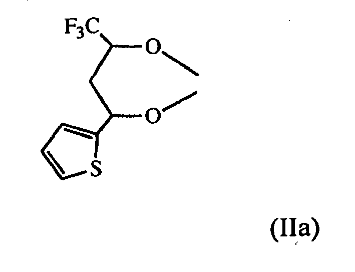

2. Verfahren nach Anspruch 1, **dadurch gekennzeichnet, daß** das Biomolekül ein Protein, ein Peptid, ein Antikörper oder eine Nukleinsäure ist.

3. Verfahren nach Anspruch 1 oder 2, **dadurch gekennzeichnet, daß** das Biomolekül an den Chelatkomplex des Elements der Seltenen Erden über eine koordinative und/oder kovalente, bevorzugt koordinative Bindung, anbindet.

4. Verfahren nach einem der vorangehenden Ansprüche, **dadurch gekennzeichnet, daß** die Bindung des Chelatkomplexes des Elements der Seltenen Erden über mindestens eine funktionelle Gruppe des Biomoleküls erfolgt, wobei die funktionelle Gruppe ausgewählt ist aus Thiolgruppen.

5. Verfahren nach einem der vorangehenden Ansprüche, **dadurch gekennzeichnet, daß** die Bindung des Biomoleküls an den Chelatkomplex des Elements der Seltenen Erden unter Ausbildung eines Konjugats aus Biomolekül und Chelatkomplex des Elements der Seltenen Erden erfolgt ("Biomolekül/Seltenerdkomplex-Konjugat").

6. Verfahren nach einem der vorangehenden Ansprüche, **dadurch gekennzeichnet, daß** der Chelatkomplex des Elements der Seltenen Erden der Formel gemäß Fig. 1B entspricht, wobei "Ln" ein Element der Seltenen Erden in der Form von Europium(III) darstellt.

7. Verfahren nach einem der vorangehenden Ansprüche, **dadurch gekennzeichnet, daß** das Konjugat aus Biomolekül und Chelatkomplex des Elements der Seltenen Erden ("Biomolekül/Seltenerdkomplex-Konjugat") unter Einwirkung von Anregungsenergie und/oder Absorption von Anregungsenergie fluoresziert.

8. Verfahren nach einem der vorangehenden Ansprüche, **dadurch gekennzeichnet, daß** das Konjugat aus Biomolekül und Chelatkomplex des Elements der Seltenen Erden ("Biomolekül/Seltenerdkomplex-Konjugat") unter Einwirkung von Anregungsenergie und/oder Absorption von Anregungsenergie detektierbare Energie in Form von Fluoreszenz freisetzt, wobei die freigesetzte und/oder emittierte Energie von der Anregungsenergie differenzierbar und/oder unterscheidbar ausgebildet ist und/oder wobei die Fluoreszenz im Bereich des sichtbaren Lichts erfolgt und/oder wobei die Anregung mit Licht einer Wellenlänge unterhalb von 400 nm erfolgt und/oder wobei die freigesetzte und/oder emittierte Energie mittels einer Detektionsvorrichtung qualitativ und/oder quantitativ erfaßt wird.

9. Verfahren nach einem der vorangehenden Ansprüche, **dadurch gekennzeichnet, daß** das Inkontaktbringen und/oder die Umsetzung von Biomolekül einerseits und Chelatkomplex des Elements der Seltenen Erden andererseits durchgeführt wird, indem zunächst eine Lösung und/oder Dispersion, vorzugsweise eine wässrige Lösung, des Biomoleküls hergestellt wird und die so hergestellte Lösung und/oder Dispersion des Biomoleküls nachfolgend mit dem Chelatkomplex des Elements der Seltenen Erden in Kontakt und zur Wechselwirkung gebracht wird.

10. Verfahren nach einem der vorangehenden Ansprüche, **dadurch gekennzeichnet, daß** bei dem Inkontaktbringen und/oder bei der Umsetzung von Biomolekül einerseits und Chelatkomplex des Elements der Seltenen Erden andererseits der Chelatkomplex des Elements der Seltenen Erden in fester Phase vorliegt, wobei der Chelatkomplex des Elements der Seltenen Erden bedarfsweise vom Substrat bei Kontakt mit dem Biomolekül freigesetzt wird und/oder wobei das feste Substrat die Wandung eines Reaktionsgefäßes, ein partikelförmiger Träger, die feste Phase einer Chromatographiereinrichtung ("HPLC-Kügelchen") oder dergleichen ist.

11. Verfahren nach einem der vorangehenden Ansprüche, **dadurch gekennzeichnet, daß** das Inkontaktbringen und/oder die Umsetzung von Biomolekül einerseits und Chelatkomplex des Elements der Seltenen Erden andererseits bei einem pH-Wert im Bereich von 3 bis 8, vorzugsweise 5,5 bis 7,5, erfolgt und/oder daß das Inkontaktbringen und/oder die Umsetzung von Biomolekül einerseits und Chelatkomplex des Elements der Seltenen Erden andererseits bei einer Temperatur im Bereich von -20 °C bis +40 °C erfolgt und/oder daß der Chelatkomplex des Elements der Seltenen Erden unter Reaktionsbedingungen nur geringfügig löslich, insbesondere nur geringfügig wasserlöslich, ausgebildet ist, insbesondere wobei die Löslichkeit geringer als die des Biomoleküls ist, vorzugsweise um den Faktor 100, insbesondere 1.000, besonders bevorzugt 10.000.

12. Verwendung mindestens eines Chelatkomplexes mindestens eines Elements der Seltenen Erden zur Markierung und/oder Identifizierung mindestens eines Biomoleküls, insbesondere eines Proteins, eines Peptids, eines Antikörpers oder einer Nukleinsäure,
wobei der Chelatkomplex des Elements der Seltenen Erden der Formel gemäß der allgemeinen Formel (I) 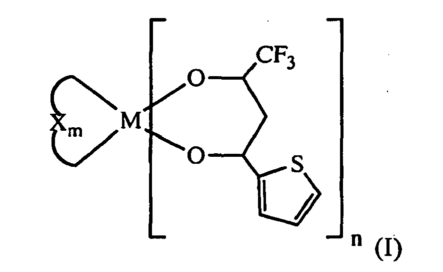 entspricht, wobei:
• "M" ein Element der Seltenen Erden in der Form von Europium(III) darstellt,
• "n" die ganze Zahl 3 bezeichnet,
• "m" die ganze Zahl 1 bezeichnet und
• "X" einen Liganden der folgenden Formel darstellt:

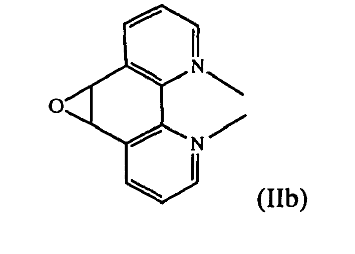

13. Verwendung mindestens eines Chelatkomplexes mindestens eines Elements der Seltenen Erden zur Fluoreszenzmarkierung oder -identifizierung mindestens eines Biomoleküls, insbesondere eines Proteins, eines Peptids, eines Antikörpers oder einer Nukleinsäure,
wobei der Chelatkomplex des Elements der Seltenen Erden der Formel gemäß der allgemeinen Formel (I) 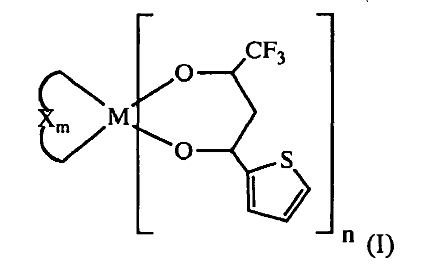 entspricht, wobei:
• "M" ein Element der Seltenen Erden in der Form von Europium(III) darstellt,
• "n" die ganze Zahl 3 bezeichnet,
• "m" die ganze Zahl 1 bezeichnet und
• "X" einen Liganden der folgenden Formel darstellt:

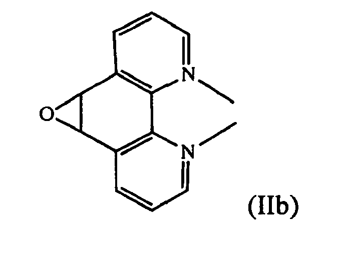

14. Kit zur Markierung und/oder Identifizierung mindestens eines Biomoleküls, umfassend mindestens ein Reaktionsgefäß und/oder einen abgegrenzten Reaktionsraum, wobei in dem Reaktionsgefäß und/oder Reaktionsraum mindestens ein Chelatkomplex mindestens eines Elements der Seltenen Erden ("Seltenerdkomplex") befindlich ist,
wobei der Chelatkomplex des Elements der Seltenen Erden der Formel gemäß der allgemeinen Formel (I) 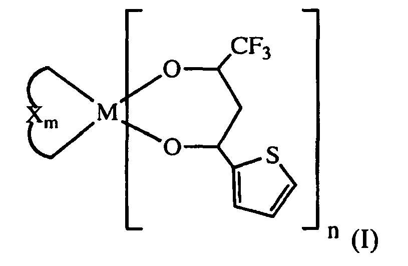 entspricht, wobei:
• "M" ein Element der Seltenen Erden in der Form von Europium(III) darstellt,
• "n" die ganze Zahl 3 bezeichnet,
• "m" die ganze Zahl 1 bezeichnet und
• "X" einen Liganden der folgenden Formel darstellt:

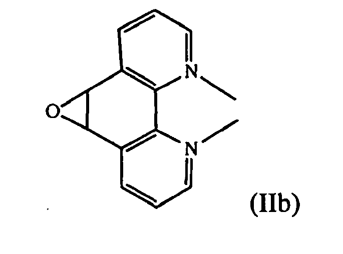

15. Biomolekül/Seltenerdkomplex-Konjugat, erhältlich durch Inkontaktbringen und/oder Umsetzung mindestens eines Biomoleküls einerseits und mindestens eines Chelatkomplexes mindestens eines Elements der Seltenen Erden ("Seltenerdkomplex") andererseits,
wobei der Chelatkomplex des Elements der Seltenen Erden der Formel gemäß der allgemeinen Formel (I) 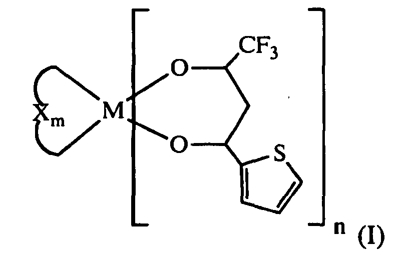 entspricht, wobei:
• "M" ein Element der Seltenen Erden in der Form von Europium(III) darstellt,
• "n" die ganze Zahl 3 bezeichnet,
• "m" die ganze Zahl 1 bezeichnet und
• "X" einen Liganden der folgenden Formel darstellt:

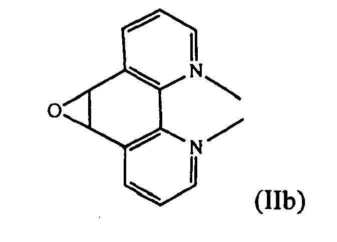

## Claims

1. A method for labelling and/or identification of at least one biomolecule,
wherein the biomolecule is brought into contact with at least one chelate complex of at least one rare earth element (rare earth complex) and caused to interact therewith, with the formation of a bond between the biomolecule on the one hand and the rare earth element chelate complex on the other hand,
wherein the contact and/or reaction of the biomolecule on the one hand and the rare earth element chelate complex on the other hand occurs in accordance with the following chemical equation: 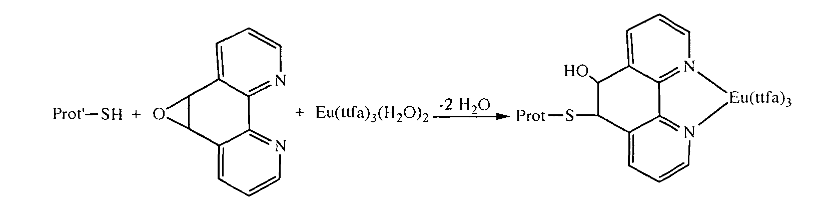 wherein:
• "Prot' " denotes a portion of the biomolecule,
• "Eu" denotes a europium(III) nucleus, and
• "ttfa" denotes a 1-(2-thenyl)-4,4,4-trifluorobutane-1,3-dionato ligand and/or "ttfa" denotes a residue with formula (IIa):

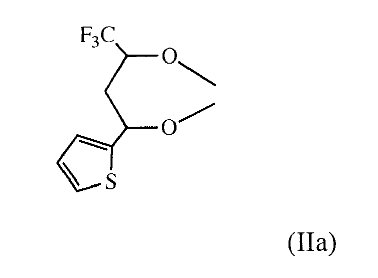

2. The method as claimed in claim 1, **characterized in that** the biomolecule is a protein, a peptide, an antibody or a nucleic acid.

3. The method as claimed in claim 1 or claim 2, **characterized in that** the biomolecule bonds to the rare earth element chelate complex via a dative and/or covalent bond, preferably a dative bond.

4. The method as claimed in one of the preceding claims, **characterized in that** bonding of the rare earth element chelate complex occurs via at least one functional group of the biomolecule, wherein the functional group is selected from thiol groups.

5. The method as claimed in one of the preceding claims, **characterized in that** bonding of the biomolecule to the rare earth element chelate complex occurs with the formation of a conjugate of the biomolecule and rare earth element chelate complex ("biomolecule/rare earth complex conjugate").

6. The method as claimed in one of the preceding claims, **characterized in that** the rare earth element chelate complex has the formula of Figure 1B, wherein "Ln" represents a rare earth element in the form of europium(III).

7. The method as claimed in one of the preceding claims, **characterized in that** the conjugate formed by the biomolecule and rare earth element chelate complex ("biomolecule/rare earth complex conjugate") fluoresces under the action of excitation energy and/or the absorption of excitation energy.

8. The method as claimed in one of the preceding claims, **characterized in that** the conjugate formed by the biomolecule and rare earth element chelate complex ("biomolecule/rare earth complex conjugate") releases detectable energy in the form of fluorescence under the action of excitation energy and/or the absorption of excitation energy, wherein the released and/or emitted energy can be distinguished from the excitation energy and/or is configured differently and/or wherein the fluorescence is in the visible light region and/or wherein the excitation is carried out using light with a wavelength of less than 400 nm and/or wherein the released and/or emitted energy is recorded qualitatively or quantitatively using a detection device.

9. The method as claimed in one of the preceding claims, **characterized in that** contact and/or reaction of the biomolecule on the one hand and the rare earth element chelate complex on the other hand is carried out by initially producing a solution and/or dispersion, preferably an aqueous solution, of the biomolecule and then bringing the solution produced thereby and/or the dispersion of the biomolecule into contact with the rare earth element chelate complex and caused to interact.

10. The method as claimed in one of the preceding claims, **characterized in that** when the biomolecule on the one hand and the rare earth element chelate complex on the other hand are brought into contact and/or reacted, the rare earth element chelate complex is in the solid phase, whereby the rare earth element chelate complex is released from the substrate on contact with the biomolecule as required and/or whereby the solid substrate is the wall of a reaction vessel, a particulate support, the solid phase of chromatographic equipment ("HPLC beads") or the like.

11. The method as claimed in one of the preceding claims, **characterized in that** contact and/or reaction of the biomolecule on the one hand and the rare earth element chelate complex on the other hand is carried out at a pH in the range 3 to 8, preferably in the range 5.5 to 7.5 and/or **in that** contact and/or reaction of the biomolecule on the one hand and the rare earth element chelate complex on the other hand is carried out at a temperature in the range -20°C to +40°C and/or **in that** under reaction conditions, the rare earth element chelate complex is only slightly soluble, in particular only slightly soluble in water, and in particular wherein the solubility is less than that of the biomolecule, preferably by a factor of 100, in particular 1000, particularly preferably 10000.

12. Use of at least one chelate complex of at least one rare earth element for labelling and/or identification of at least one biomolecule, in particular a protein, a peptide, an antibody or a nucleic acid,
wherein the formula for the rare earth element chelate complex is in accordance with general formula (I) 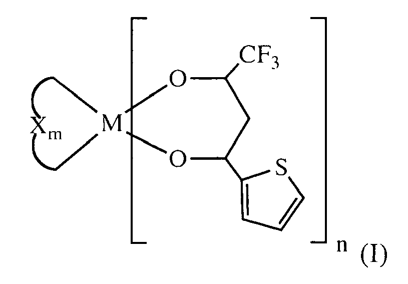 wherein:
• "M" is a rare earth element in the form of europium(III),
• "n" is the integer 3,
• "m" is the integer 1, and
• "X" is a ligand with the following formula:

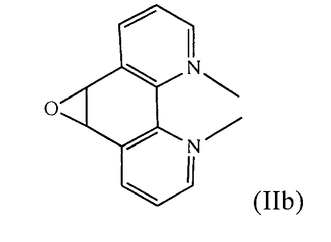

13. Use of at least one chelate complex of at least one rare earth element for fluorescence labelling and/or identification of at least one biomolecule, in particular a protein, a peptide, an antibody or a nucleic acid,
wherein the formula for the rare earth element chelate complex is in accordance with general formula (I) 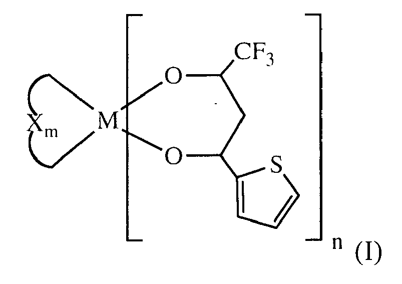 wherein:
• "M" is a rare earth element in the form of europium(III),
• "n" is the integer 3,
• "m" is the integer 1, and
• "X" is a ligand with the following formula:

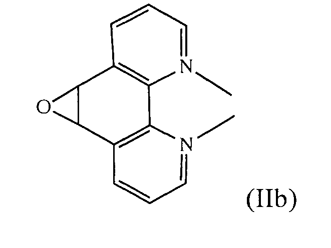

14. A kit for labelling and/or identification of at least one biomolecule, comprising at least one reaction vessel and/or a distinct reaction volume, wherein the reaction vessel and/or reaction volume contains at least one rare earth element chelate complex ("rare earth complex"),
wherein the formula for the rare earth element chelate complex is in accordance with general formula (I) 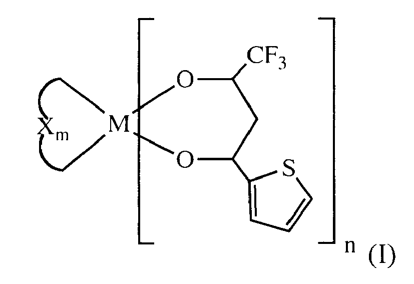 wherein:
• "M" is a rare earth element in the form of europium(III),
• "n" is the integer 3,
• "m" is the integer 1, and
• "X" is a ligand with the following formula:

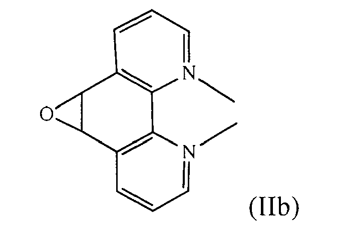

15. A biomolecule/rare earth complex conjugate obtainable by contact and/or reaction of at least one biomolecule on the one hand and at least one rare earth element chelate complex ("rare earth complex") on the other hand,
wherein the formula for the rare earth element chelate complex is in accordance with general formula (I) 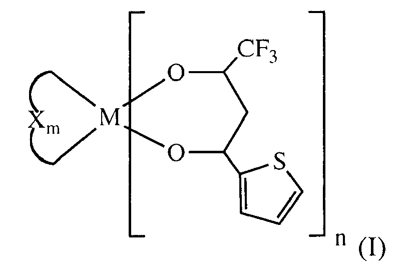 wherein:
• "M" is a rare earth element in the form of europium(III),
• "n" is the integer 3,
• "m" is the integer 1, and
• "X" is a ligand with the following formula:

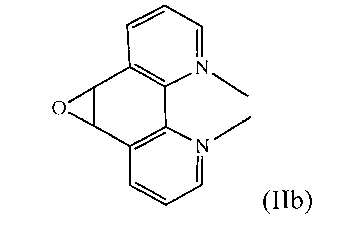

## Revendications

1. Procédé de marquage et/ou d'identification d'au moins une biomolécule, lors duquel on met la molécule en contact avec au moins un complexe de chélate d'au moins un élément de la terre rare (« complexe de terre rare ») et de ce fait, en formant une liaison entre la biomolécule d'une part et le complexe de chélate de l'élément de la terre rare d'autre part, on l'amène en interaction,
la mise en contact et/ou la transformation de la biomolécule d'une part et du complexe de chélate de l'élément de la terre rare d'autre part s'effectuant selon l'équation de réaction suivante : 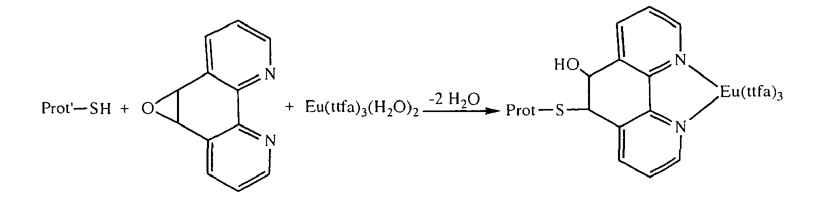 dans laquelle :
• « Prot » désigne une partie de biomolécule,
• « Eu » désigne un noyau d'europium et
• « ufa » désigne un ligand (1-(2-thényl)-4,4,4,trifluorobutane-1,3-dionato et/ou « ttfa » désigne un radical de la formule IIa) :

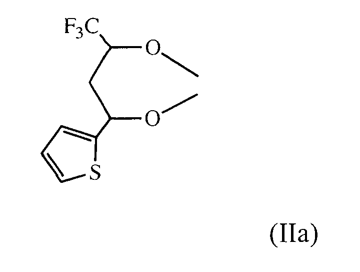

2. Procédé selon la revendication 1, **caractérisé en ce que** la biomolécule est une protéine, un peptide, un anticorps ou un acide nucléique.

3. Procédé selon la revendication 1 ou la revendication 2, **caractérisé en ce que** la biomolécule se fixe sur le complexe de chélate de l'élément de la terre rare par l'intermédiaire d'une liaison coordonnée et/ou covalente, de préférence d'une liaison coordonnée.

4. Procédé selon l'une quelconque des revendications précédentes, **caractérisé en ce que** la liaison du complexe de chélate de l'élément de la terre rare s'effectue par l'intermédiaire d'au moins un groupe fonctionnel de la biomolécule, le groupe fonctionnel étant choisi parmi des groupes thiol.

5. Procédé selon l'une quelconque des revendications précédentes, **caractérisé en ce que** la liaison de la biomolécule sur le complexe de chélate de l'élément de la terre rare s'effectue sous formation d'un conjugué de la biomolécule et du complexe de chélate de la terre rare (« conjugué de la biomolécule/du complexe de terre rare »).

6. Procédé selon l'une quelconque des revendications précédentes, **caractérisé en ce que** le complexe de chélate correspond à l'élément de la terre rare de la formule selon la figure 1B, « Ln » représentant un élément de la terre rare sous la forme d'europium(III).

7. Procédé selon l'une quelconque des revendications précédentes, **caractérisé en ce que** le conjugué de la biomolécule et du complexe de chélate de l'élément de la terre rare (« conjugué de la biomolécule/du complexe de terre rare ») entre en fluorescence sous l'effet d'énergie d'excitation et/ou sous absorption d'énergie d'excitation.

8. Procédé selon l'une quelconque des revendications précédentes, **caractérisé en ce que** sous l'effet d'énergie d'excitation et/ou sous absorption d'énergie d'excitation, le conjugué de la biomolécule et du complexe de chélate de l'élément de la terre rare (« conjugué de la biomolécule/du complexe de terre rare ») libère de l'énergie détectable sous le forme de fluorescence, l'énergie libérée et/ou émise étant conçue de manière à pouvoir être différenciée et/ou distinguée et/ou la fluorescence s'effectuant dans la plage de la lumière visible et/ou l'excitation s'effectuant avec de la lumière d'une longueur d'onde inférieure à 400 nm et/ou l'énergie libérée et/ou émise étant détectée au niveau qualitatif et/ou quantitatif au moyen d'un dispositif de détection.

9. Procédé selon l'une quelconque des revendications précédentes, **caractérisé en ce qu'**on réalise la mise en contact et/ou la transformation d'une biomolécule d'une part et d'un complexe de chélate de l'élément de la terre rare d'autre part **en ce qu'**on produit d'abord une solution et/ou dispersion, de préférence une solution aqueuse de la biomolécule et **en ce qu'**on met ensuite la solution et/ou la dispersion ainsi produite en contact avec le complexe de chélate de l'élément de la terre rare et on l'amène en interaction.

10. Procédé selon l'une quelconque des revendications précédentes, **caractérisé en ce que** lors de la mise en contact et/ou de la transformation de la biomolécule d'une part et du complexe de chélate de l'élément de la terre rare d'autre part, le complexe de chélate de l'élément de la terre rare se présente en phase solide, le complexe de chélate de l'élément de la terre rare étant libéré en cas de besoin par le substrat, lors du contact avec la biomolécule et/ou le substrat solide étant la paroi d'un récipient de réaction, un support en forme de particule, la phase solide d'une installation de chromatographie (perles de HPLC) ou similaires.

11. Procédé selon l'une quelconque des revendications précédentes, **caractérisé en ce que** la mise en contact et/ou la transformation de la biomolécule d'une part et du complexe de chélate de l'élément de la terre rare d'autre part s'effectue à une valeur pH de l'ordre de 3 à 8, de préférence de 5,5 à 7,5 et/ou **en ce que** la mise en contact et/ou la transformation de la biomolécule d'une part et du complexe de chélate de l'élément de la terre rare d'autre part s'effectue à une température de l'ordre de -20°c à +40°c et/ou **en ce que** le complexe de chélate de l'élément de la terre rare est conçu pour n'être que faiblement soluble, notamment faiblement hydrosoluble, la solubilité étant notamment inférieure à celle de la biomolécule, de préférence de la valeur du coefficient 100, notamment 1.000, de manière particulièrement préférée 10.000.

12. Utilisation d'au moins un complexe de chélate d'au moins un élément de la terre rare pour le marquage et/ou l'identification d'au moins une biomolécule, notamment d'une protéine, d'un peptide, d'un anticorps ou d'un acide nucléique,
le complexe de chélate de l'élément de la terre rare correspondant à la formule selon la formule générale (I) 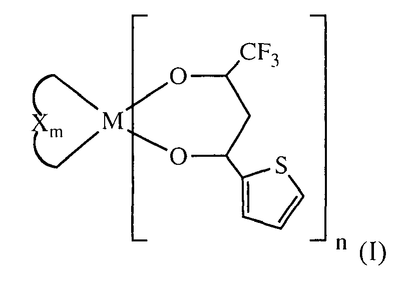 dans laquelle
• « M » représente un élément de la terre rare sous la forme d'europium(III),
• « n » désigne le nombre entier 3,
• « m » représente le nombre entier 1 et
• « X » représente un ligand de la formule suivante :

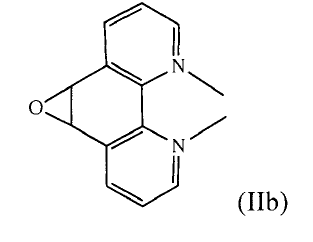

13. Utilisation d'au moins un complexe de chélate d'au moins un élément de la terre rare pour le marquage et/ou l'identification par fluorescence d'au moins une biomolécule, notamment d'une protéine, d'un peptide, d'un anticorps ou d'un acide nucléique,
le complexe de chélate de l'élément de la terre rare correspondant à la formule selon la formule générale (I), 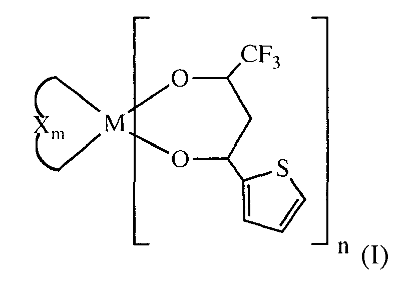 dans laquelle
• « M » représente un élément de la terre rare sous la forme d'europium(III),
• « n » désigne le nombre entier 3,
• « m » représente le nombre entier 1 et
• « X » représente un ligand de la formule suivante :

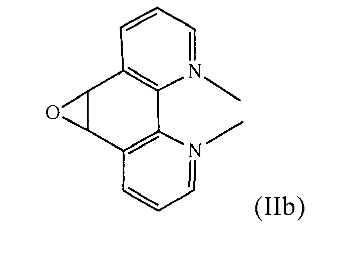

14. Kit pour le marquage et/ou l'identification d'au moins une biomolécule, comprenant au moins un récipient de réaction et/ou un espace de réaction délimité, dans le récipient de réaction et/ou dans l'espace de réaction se trouvant au moins un complexe de chélate d'au moins un élément de la terre rare (complexe de terre rare),
le complexe de chélate de l'élément de la terre rare correspondant à la formule selon la formule générale (I) 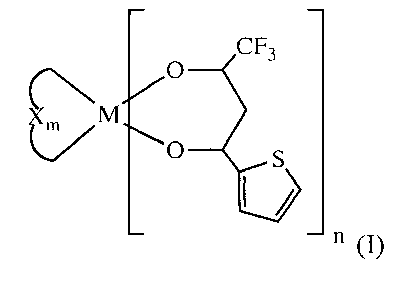 dans laquelle
• « M » représente un élément de la terre rare sous la forme d'europium(III),
• « n » désigne le nombre entier 3,
• « m » représente le nombre entier 1 et
• « X » représente un ligand de la formule suivante :

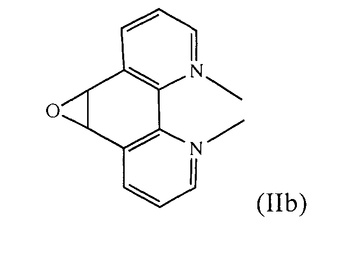

15. Conjugué d'une biomolécule/d'un complexe de terre rare, pouvant être obtenu par mise en contact et/ou transformation d'au moins une biomolécule d'une part et d'au moins un complexe de chélate d'au moins un élément de la terre rare (« complexe de terre rare ») d'autre part,
le complexe de chélate de l'élément de la terre rare correspondant à la formule selon la formule générale (I) 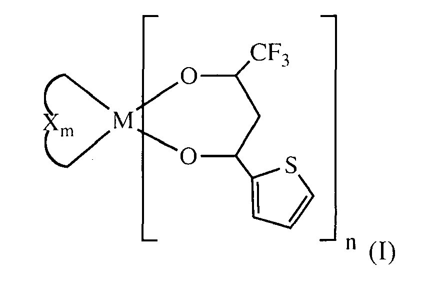 dans laquelle
• « M » représente un élément de la terre rare sous la forme d'europium(III),
• « n » désigne le nombre entier 3,
• « m » représente le nombre entier 1 et
• « X » représente un ligand de la formule suivante :
